# EUROPÄISCHE PATENTSCHRIFT

(11) **EP 1 662 996 B1**
(45) Veröffentlichungstag und Bekanntmachung des Hinweises auf die Patenterteilung: **19.11.2014**
(21) Anmeldenummer: 04764809.2
(22) Anmeldetag: 03.09.2004
(51) Int. Cl.: A61B 5/087

(54) **ERFASSUNGSGERÄT SOWIE VERFAHREN ZUR OBSERVATION SCHLAFBEZOGENER ATMUNGSSTÖRUNGEN**
DETECTION APPLIANCE AND METHOD FOR OBSERVING SLEEP-RELATED BREATHING DISORDERS
DISPOSITIF DE DETECTION ET PROCEDE D'OBSERVATION DE TROUBLES RESPIRATOIRES LIES AU SOMMEIL

(30) Priorität: 03.09.2003 DE 10340654
(43) Veröffentlichungstag der Anmeldung: 07.06.2006
(73) Patentinhaber: ResMed R&D Germany GmbH, 82152 Martinsried (DE)
(72) Erfinder: MADAUS, Stefan, 82166 Gräfelfing (DE); STAUFFENBERG, Caspar Graf von, 82131 Gauting (DE); VÖGELE, Harald, 82131 Gauting (DE); HEIDMANN, Dieter, Castle Hill, NSW 2154 (AU); KLAUS, Dieter, 79689 Maulburg (DE)
(74) Vertreter: Vossius & Partner
(86) Internationale Anmeldenummer: PCT/EP2004/009857
(87) Internationale Veröffentlichungsnummer: WO 2005/023109

(56) Entgegenhaltungen:
- WO-A-02/22017
- WO-A-93/09834
- WO-A-03/061471
- DE-A- 10 164 445
- US-A- 5 458 137

## Beschreibung

Die Erfindung betrifft ein Erfassungsgerät sowie ein Verfahren zur Erfassung und Auswertung eines hinsichtlich der Atmung einer schlafenden Person indikativen Messsignals im Zusammenhang mit der Observation schlafbezogener Atmungsstörungen. Weiterhin betrifft die Erfindung auch Instrumente zum Abgriff von hinsichtlich der Atmung eines Patienten indikativen Signalen.

Die WO 93/09834 A1 diskutiert Systeme und Verfahren zur Behandlung obstruktiver Schlaf-Apnoen, bei denen ein adaptives Kontroll-System genutzt wird, um in der Luftröhre Luftdruck bereitzustellen.

Zur Untersuchung schlafbezogener Atmungsstörungen sind sog. Polysomnographiegeräte bekannt, die üblicherweise über mehrere Messkanäle zur Erfassung verschiedener Messsignale für physiologische Zustandsgrößen eines Patienten, verfügen. Wie aus der auf die Anmelderin zurückgehenden Patentschrift DE 101 64 445 A1 hervorgeht, ist es bekannt, zur Diagnose eines Patienten im Hinblick auf dessen Atmungseigenschaften während einer Schlafphase EKG- und Blutdrucksignale zu erfassen und diese gemeinsam mit Signalen, die die Atmungstätigkeit des Patienten beschreiben, aufzuzeichnen. Jene die Atmungstätigkeit des Patienten beschreibenden Signale können über sog. Thermistoren oder auch über Pneumotachographen generiert werden. Die erfassten Signale können in zeitlicher Zuordnung zueinander visualisiert und im Rahmen einer fachärztlichen Betrachtung ausgewertet werden. Auf Grundlage der fachärztlichen Auswertung kann festgestellt werden, ob etwaigen Atmungsstörungen durch Zufuhr des Atemgases auf einem erhöhten Druckniveau (CPAP-Therapie) vorgebeugt werden kann. Geeignete Therapieparameter können ebenfalls im Rahmen der fachärztlichen Auswertung ermittelt werden.

Bei der Untersuchung von Personen mit Anzeichen auf schlafbezogene Atmungsstörungen führt die Betrachtung der generierten Messsignale in der Praxis unter Umständen zu unterschiedlichen Befunden insbesondere hinsichtlich Art- und Schweregrad obstruktionsrelevanter Beeinträchtigungen der Atemwege sowie hinsichtlich der Bewertung des allgemeinen physiologischen Zustands des Patienten. Bei unzureichender Bewertung eines Krankheitsbildes besteht das Problem, dass ein ggf. erforderlicher Therapiebedarf nicht erkannt, oder für eine Therapie Rahmenbedingungen gewählt werden, die den tatsächlichen physiologischen Bedürfnissen nicht hinreichend Rechnung tragen, oder zumindest den Therapiekomfort einschränken.

Der Entschluss, sich zur Untersuchung auf etwaige schlafbezogene Atmungsstörungen in ein Schlaflabor zu begeben, wird vom Betroffenen häufig erst getroffen, wenn Folgeerscheinungen des Krankheitsbildes OSA das Befinden desselben bereits erheblich belasten. Die durch OSA verursachte Belastung des Betroffenen erschwert im fortgeschrittenen Stadium der Erkrankung die präzise Diagnose derselben.

Der Erfindung liegt die Aufgabe zugrunde, Lösungen bereitzustellen, die es ermöglichen, eine besonders aussagefähige Untersuchung hinsichtlich des Auftretens schlafbezogener Atmungsstörungen, insbesondere in der gewohnten Umgebung des Betroffenen durchzuführen.

Diese Aufgabe wird durch das mobile Erfassungsgeräts der Ansprüche 1 bis 17 und das Verfahren der Ansprüche 18 bis 22 gelöst.

Ein mobiles Erfassungsgerät wird diskutiert. Dieses Erfassüngsgerät hat eine Sensoreinrichtung zur Erfassung eines hinsichtlich eines nasalen Atemgasstromes indikativen Nasalstromsignales, und/oder eines oralen Atemgasstromes indikativen Atemgasstromsignals; und eine unter Einschluss einer Speichereinrichtung gebildeten elektronischen Datenverarbeitungseinrichtung zur Verarbeitung der hinsichtlich des zeitlichen Verlaufs der nasalen bzw. der oralen Atemung indikativen Atemstromsignale, wobei die Datenverarbeitungseinrichtung derart konfiguriert ist, dass diese eine Speicherung von hinsichtlich des zeitlichen Verlaufs der Atemstromsignale indikativen Daten veranlasst.

Dadurch wird es auf vorteilhafte Weise möglich, mittels einer zur Selbstapplikation geeigneten Erfassungseinrichtung im häuslichen Bereich, d.h. in der gewohnten

Umgebung des Betroffenen hinsichtlich der Atmung während einer Schlafphase charakteristische Merkmale in einer Weise zu erfassen, die es gestattet, den physiologischen Zustand des Betroffenen mit besonders hoher Aussagekräftigkeit zu bestimmen. Auf Grundlage einer standardisierten, rechnerbasierten Auswertung der Messergebnisse kann nachfolgend beurteilt werden, ob - und ggf. mit welchem Schweregrad das Krankheitsbild OSA vorliegt und ob eine tiefergehende Untersuchung durch ein Schlaflabor zu empfehlen ist.

Die Datenverarbeitungseinrichtung, oder ein dieser vorgelagerter Signalübertragungsschaltkreis ist vorzugsweise derart konfiguriert, dass eine Prüfung ermöglicht ist, ob das jeweils erfasste Atemstromsignal vorgegebene Signaiqualitätskriterien erfüllt. Für den Fall, dass das anliegende Signal bestimmte Kriterien nicht erfüllt, ist es möglich die Signalaufzeichnung zu unterdrücken, oder auch Aufzeichnungen vorzunehmen, aus welchen die zeitliche Lage von als unzulässig klassifizierten Signalen hervorgeht.

Die Datenverarbeitungseinrichtung ist vorzugsweise derart ausgebildet, dass diese Zugriff zu einer Zeitgebereinrichtung hat, und dass die hinsichtlich des jeweiligen Atemgasstroms indikativen Daten mit einer Zeitinformation verknüpft aufgezeichnet werden.

In Verbindung mit jener Datenverarbeitungseinrichtung ist vorzugsweise ein Datenkompressionssystem verwirklicht, durch welches die erfassten, sich zeitlich ändernden Signale komprimiert aufgezeichnet werden können.

Die Datenverarbeitungseinrichtung ist weiterhin vorzugsweise derart konfiguriert, dass der Aufzeichnungsvorgang durch einen anwenderseitig veranlassten Schaltimpuls ausgelöst wird. Es ist möglich, den Schaltimpuls in Abhängigkeit davon zu generieren, ob ein Schalttaster über eine vorgegebene Mindestdauer von z.B. 3 Sekunden betätigt wurde.

Die Datenverarbeitungseinrichtung kann derart ausgebildet sein, dass diese die Datenspeicherung veranlasst, wenn die anliegenden Atemstromsignale ein bestimmtes Kriterium z.B. ein vorgegebenes Periodizitätskriterium erfüllen.

Vorzugsweise umfasst das Erfassungsgerät einen ersten Druckmessanschluss an welchen eine Messkanüle, ein Messkanülenpaar, oder ein Messkanülenbündel anschließbar ist. Die erste Messkanüle kann mit einer Nasaldruckmessbrilleneinrichtung verbunden sein, zum Abgriff eines Staudrucksignals aus dem, aus den Nasenöffnungen abströmenden Atemgasstrom. Die zweite Messkanüle kann derart an den Anwender herangeführt werden, dass über diese ein hinsichtlich des über den Mund des Anwenders ggf. fließenden Gasstroms indikatives Signal erhebbar ist.

Es ist möglich, das Erfassungsgerät mit einem zweiten ebenfalls der Erfassung der nasalen Atmung dienenden Druckmessanschluss auszustatten, zur Erfassung eines zweiten Nasalatemgasstromsignals. Durch die Möglichkeit der Erfassung von zwei Druckmessignalen wird es möglich, das Erfassungsgerät derart zu betreiben, dass dieses für die linke und rechte Nasenöffnung jeweils indikative Atemstromsignale erfassen kann.

Vorzugsweise umfasst das Erfassungsgerät eine Einrichtung zur Erfassung der Thoraxweitung. Die Einrichtung zur Erfassung der Thoraxweitung kann ein Bandelement umfassen, das um den Thoraxbereich des Anwenders herumführbar ist. Es ist möglich, dieses Bandelement derart auszugestalten, dass aus diesem ein hinsichtlich der Banddehnung, oder Bandbelastung indikatives Signal ableitbar ist (z.B. Änderung des elektrischen Widerstands eines eingebetteten Leiters). Es ist auch möglich, an dem Erfassungsgerät Vorkehrungen zu treffen, durch welche die Bandbelastung erfasst werden kann. Insbesondere ist es möglich, an dem Erfassungsgerät eine Schlaufeneinrichtung vorzusehen, durch welche die Bandweitung, oder Bandkräfte erfasst werden können. Es ist auch möglich, an dem Erfassungsgerät Druck- oder Krafterfassungsstrukturen vorzusehen, durch welche die durch ein über diese Krafterfassungsstrukturen geführtes Band aufgebrachten Kräfte erfasst werden können. Das Band zur Erfassung der Thoraxweitung kann zugleich der Befestigung des Erfassungsgeräts am Anwender dienen.

Vorzugsweise ist der Aufzeichnungsvorgang durch ein anwenderseitig veranlassbares Schaltsignal beendbar. Dieses Schaltsignal kann insbesondere über den vorangehend zum Einschalten des Geräts verwendeten Schalter, insbesondere Druckknopf, generiert werden. Es ist weiterhin auch möglich, das Erfassungsgerät derart zu konfigurieren, dass der Aufzeichnungsvorgang durch Erfüllung eines Zeitkriteriums beendbar ist. Insbesondere ist es möglich, den Aufzeichnungsvorgang zu beenden, wenn dieser eine vorgegebene Aufzeichnungsdauer von z.B. 9,5 h erreicht hat.

Weiterhin ist es möglich, den Aufzeichnungsvorgang schaltungsgesteuert zu beenden, wenn das anliegende Atemstromsignal über ein bestimmtes Abschaltzeitfenster ein bestimmtes Abschaltkriterium erfüllt. Es ist möglich, die Aufzeichnungskapazität auf eine bestimmte Anzahl von Aufzeichnungen, insbesondere auf zwei Aufzeichnungen zu begrenzen.

Gemäß einem besonderen Aspekt der vorliegenden Erfindung ist das Erfassungsgerät mit einer Schnittstelleneinrichtung versehen, zur Übertragung der aufgezeichneten Daten auf ein externes Auswertungssystem. Diese Schnittstelleneinrichtung ist vorzugsweise als USB-Schnittstelle oder auch als Infrarot-Schnittstelle ausgebildet.

In besonders vorteilhafter Weise ist es auch möglich, das Erfassungsgerät derart zu gestalten, dass die Speichereinrichtung austauschbar in dem Erfassungsgerät aufgenommen ist. Die Speichereinrichtung kann hierzu als Karte oder insbesondere USB-Flashstick ausgebildet sein. Es ist möglich, durch einen vorab auf der Speichereinrichtung veranlassten Eintrag personenspezifische Daten auf dem Speichermedium aufzuzeichnen. Auf Grundlage dieser Vorab-Aufzeichnungen ist es möglich, eine Vorkonfiguration des Erfassungsgerätes vorzunehmen, oder eine zutreffende Zuordnung der erfasten Daten zu dem Anwender sicherzustellen.

Die Konfiguration der Datenverarbeitungseinrichtung ist vorzugsweise durch ein Datenverarbeitungsprogramm festgelegt, wobei dieses Datenverarbeitungsprogramm vorzugsweise veränderbar oder wechselbar ist. Die Abbildung des Datenverarbeitungsprogramms im Bereich des Erfassungsgerätes kann über die vorgenannten Schnittstelleneinrichtungen, zusätzliche Schnittstelleneinrichtungen, oder auch das Speichermedium erfolgen.

An dem Erfassungsgerät sind neben vorzugsweise intuitiv leicht zu bedienenden Schaltereinrichtung auch Anzeigeeinrichtungen vorgesehen, zur Anzeige der Betriebsbereitschaft oder des Funktionszustands des Erfassungsgerätes. Es ist möglich, die Aufzeichnungsbereitschaft des Erfassungsgerätes durch periodisches Aufblinken einer Signaldiode zu signalisieren.

Das Erfassungsgerät umfasst vorzugsweise eine Spannungsversorgungseinrichtung, die beispielsweise durch eine Batterieeinrichtung gebildet sein kann. Vorzugsweise ist die Batterieeinrichtung möglichst kompakt ausgebildet, so dass das Erfassungsgerät flach und kleinbauend ausgeführt werden kann und nur ein geringes Eigengewicht aufweist.

Die Datenverarbeitungseinrichtung ist vorzugsweise mit einer Kalibriereinrichtung gekoppelt, zur Kalibrierung des Atemstromsignals. Die Kallibriereinrichtung kann derart ausgebildet sein, dass diese eine selbsttätige Anpassung des Systems an die erfassten Signalpegel vornimmt.

Gemäß einem besonderen Aspekt der vorliegenden Erfindung ist das Erfassungsgerät derart aufgebaut, dass dieses eine Strukturkomponente aufweist, die zu einem Wiedergabegerät kompatibel ist, das in seinem Aufbau einem Game-Boy entspricht.

Es ist möglich, das Erfassungsgerät derart auszugestalten, dass zumindest ein Abschnitt desselben in einen Einschubschacht eines Game-Boy einführbar ist.

Gemäß einem besonderen Aspekt der vorliegenden Erfindung ist das Erfassungsgerät derart ausgebildet, dass dieses ein Basismodul und ein mit diesem koppelbares Aufzeichnungstransfermodul umfasst.

Vorzugsweise ist das Aufzeichnungsmodul als jene Game-Boy kompatible Struktur ausgebildet. Hierdurch wird es möglich, die aufgezeichneten Daten über eine intuitiv leicht verständliche Benutzeroberfläche über ein handelsübliches Nutzerendgerät zu visualisieren und ggf. im Hinblick auf ausgewählte Eigenschaften zu verarbeiten.

Über das Basismodul erfolgt vorzugsweise die Spannungsversorgung, sowie die Konvertierung des Drucksignales. Das Basismodul umfasst hierzu vorzugsweise einen Batterieschacht und einen Drucksensor.

Das Aufzeichnungsrnodul umfasst vorzugsweise eine Datenverarbeitungseinrichtung die derart konfiguriert ist, dass diese eine Aufzeichnung von hinsichtlich des zeitlichen Verlaufs der Atmung indikativen Signalen auf einer Speichereinrichtung veranlasst. Das Aufzeichnungsmodul kann mit einer Schnittstelleneinrichtung versehen sein, zum Auslesen der aufgezeichneten Daten. Es ist möglich, die Speichereinrichtung lösbar mit dem Aufzeichnungsmodul zu koppeln, so dass es möglich wird, die Speichereinrichtung von dem Aufzeichnungsmodul zu trennen und einem anderweitigen System zur weiteren Auswertung und Visualisierung zuzuführen.

Die Erfassung des Atmungssignales kann alternativ zu einer Erfassung unter Verwendung einer Nasalkanüleneinrichtung auch über anderweitige Messmittel erfolgen.

Ebenso wird ein Verfahren diskutiert. Es handelt sich um ein Verfahren zur Bereitstellung eines hinsichtlich des physiologischen Zustands indikativen Auswertungsresultats auf der Grundlage von Messsignalen die mit der Atmung einer Person im Zusammenhang stehen, wobei aus den genannten Messsignalen unter Heranziehung mehrerer, Auswertungssysteme, Auswerturtgsmerkmale generiert werden und im Rahmen eines hierauf basierenden Resultat-Generierungsschrittes wenigstens ein Auswertungsresultat generiert wird, indem die Auswertungsmerkmale einer verknüpfenden Betrachtung unterzogen werden, wobei die Messsignale im Rahmen einer der Auswertung vorangehenden Signalerfassungsphase ambulant durch ein seitens des Betroffenen applizierbares Erfassungsgerät erfasst werden.

Dadurch wird es auf vorteilhafte Weise möglich, im Rahmen einer ca. 6 bis 8 Stunden andauernden anwenderseitig ambulant durchgeführten Signalerhebung eine Datenmenge zu schaffen, aus welcher Auswertungsmerkmale generiert werden können, aus weichen mit hoher Aussagefähigkeit in einer standardisiert wiederholbaren Weise Auswertungsresultate gewonnen werden können, die in vorteilhafter Weise einer nachfolgenden Diagnose zugrundegelegt werden können und damit eine standardisierten Vorabbewertung ermöglichen.

Die verknüpfende Betrachtung der für die einzelnen Atemzüge ermittelten Atemzugseigenschaften kann sich über ein Zeitfenster erstrecken das beispielsweise eine vorgegebene z.B. 30 Atemzüge oder adaptiv optimierte Anzahl von Atemzügen überblickt. Insbesondere für die Beurteilung des physiologischen Zustands des Anwenders, beispielsweise als Grundlage für eine ärztliche Diagnose, ist es auch möglich, bestimmte Verknüpfungsoperationen, für schlafphasenbezogene Perioden, ausgewählte Zeitabschnitte oder auch die gesamte Messung umfassende Zeitfenster durchzuführen. Gemäß einer besonders bevorzugten Ausführungsform der Erfindung werden auf Grundlage von Verknüpfungsoperationen Rohdaten und/oder Zwischenergebnisse selektiert die eine besonders zuverlässige Generierung von charakteristischen Kennwerten insbes. Indizes ermöglichen.

Gemäß einer besonders bevorzugten Ausführungsform der Erfindung erfolgt auf der Grundlage der verknüpfenden Betrachtung eine physiologische Typisierung des gegebenenfalls vorhandenen Krankheitsbildes der untersuchten Person.

Gemäß einer besonders bevorzugten Ausführungsform der Erfindung werden die Auswertungsmerkmale auf Grundlage von Korrelationskriterien insbesondere statistischen Auswertungssystemen generiert, durch welche beispielsweise Gemeinsamkeiten mit vorangegangenen Atemzügen, oder vorzugsweise adaptiv optimierten Bezugskriterien z.B. zu Referenzatemzügen bewertet werden. Die Korrelationskriterien können insbesondere auch auf die erste und/oder zweite Ableitung des erfassten Atemgasstromes angewendet werden. Eine Generierung der für den jeweiligen Atemzug charakteristischen Merkmale kann unter Heranziehung statistischer Methoden erfolgen. Auch die verknüpfende Betrachtung der für jeden Atemzug ermittelten Eigenschaften kann unter Heranziehung statistischer Methoden erfolgen.

Auf Grundlage der für jeden Atemzug oder auch bestimmte Atemzugsfolgen generierten Auswertungsmerkmale kann sukzessive ein Merkmalsarray aufgefüllt werden, wobei dieses Merkmalsfeld zumindest für ausgewählte Auswertungsmerkmale ein Zeitfenster beschreibt, das wenigstens so groß ist, wie das kleinste, zur verknüpfenden Betrachtung der Auswertungsmerkmale vorgesehene Verknüpfungszeitfenster.

Die Auswertungsmerkmale werden gemäß einer besonders bevorzugten Ausführungsform der Erfindung derart generiert, dass sich unter diesen z.B. Auswertungsmerkmale befinden, die Angaben zur Dauer eines Atemzuges und/oder z.B. hinsichtlich als normal anzusehender Atemzüge charakteristische Angaben enthalten. Auf Grundlage dieser Auswertungsmerkmale wird es möglich, die zeitliche Länge von Perioden mit normaler Atmung, im Rahmen der verknüpfenden Betrachtung zu bestimmen.

Weiterhin werden die Auswertungsmerkmale in vorteilhafter Weise auch derart generiert, dass diese das Auftreten etwaiger Flusslimationserscheinungen in einzelnen Atemzügen vorzugsweise auch bestimmte, hinsichtlich der Flusslimitation repräsentative Angaben enthalten. Auf Grundlage einer verknüpfenden Betrachtung der für derartige flusslimitierte Atemzüge erfassten Auswertungsmerkmale wird es möglich, die zeitliche Dauer bestimmter Eigenschaften von zumindest teilweise flusslimitierten Atmungssequenzen zu beschreiben.

Für Perioden in denen keine Atmungstätigkeit erfasst wird, können ebenfalls Auswertungsmerkmale generiert werden anhand welcher die Phasenlänge etwaiger Apnoesequenzen und/oder auch für Eigenschaften dieser Apnoephase charakteristische Merkmale im Rahmen der verknüpfenden Betrachtung generiert werden können. Diese Auswertungsmerkmale enthalten vorzugsweise Informationen hinsichtlich der Art der Apnoephasen z.B. darüber, ob die Apnoephase als zentrale, als obstruktive oder als Kombination (gemischte Apnoephase) einzustufen ist.

Derartige Auswertungsmerkmale werden gemäß einer besonders bevorzugten Ausführungsform der Erfindung auch für Schnarchphasen, für Phasen mit Cheyne-Stokes Atmung und Hypoventilationphasen generiert.

In den Auswertungsmerkmalen sind vorzugsweise auch Angaben bzw. Informationen enthalten aus welchen die Körperposition, die Kopfposition und vorzugsweise auch der Halsverdrehungsgrad des Anwender ableitbar ist. Bereits in den Auswertungsmerkmalen können schlafphasenindikative Angaben enthalten sein.

Die generierten Auswertungsmerkmale werden vorzugsweise unter Zuordnung zu dem erfassten Atemzug bzw. unter Berücksichtigung ihrer zeitlichen Lage gespeichert. D.h. die generierten Auswertungsmerkmale sind einem definierten Zeitfenster - im Falle normaler Atmung dem jeweiligen Atemzug zuordbar.

Weiterhin ist es möglich, im Rahmen der verknüpfenden Betrachtung, einen Schnarchindex zu generieren.

Weiterhin ist es möglich im Rahmen der verknüpfenden Betrachtung einen Schlafphasenindex zu generieren. Es ist in Verbindung mit der Atemphasenbetrachtung möglich zwischen inspiratorischem (obstruktionsrelevantem) und expiratorischem (minderrelevantem) Schnarchen zu unterscheiden. Weiterhin ist es möglich im Rahmen der verknüpfenden Betrachtung einen periodischen Atmungsindex zu generieren. Weiterhin ist es möglich im Rahmen der verknüpfenden Betrachtung einen Atemvolumenindex zu generieren.

Die Messung des Atemgasstromes kann bei Umgebungsdruck oder auch unter definiert verändertem Atemgasdruck erfolgen.

Zumindest ein Teil der Auswertungsmerkmale wird vorzugsweise unter Berücksichtigung der ersten oder zweiten Ableitung des zeitlichen Verlaufs des Atemgasstromes generiert.

Weiterhin wird eine Vorrichtung zur Durchführung des vorangehend beschriebenen Verfahrens diskutiert, wobei diese Vorrichtung eine Messsignaleingabeeinrichtung und eine Rechnereinrichtung zur Bereitstellung mehrerer Auswertungssysteme umfasst, wobei aus den genannten Messsignalen durch die Auswertungsysteme, Auswertungsmerkmale generiert werden und im Rahmen eines hierauf basierenden Resultat-Generierungsschrittes wenigstens ein Auswertungsresultat generiert wird indem die Rechnereinrichtung derart konfiguriert ist, dass diese die Auswertungsmerkmale einer verknüpfenden Betrachtung unterzieht.

Im Rahmen der Erfassung der Atmungstätigkeit des Anwender auf Grundlage von hinsichtlich des Atemgasvolumenstromes indikativen Daten ist es möglich, einzelne Atemzüge als solche zu erkennen. Anfang und Ende der In- und Expirationsphasen des Atemzuges können beispielsweise in Verbindung mit einer Untersuchung der ersten und zweiten Ableitung des Atemgasstromsignals sowie auch unter Berücksichtigung des möglichen Atemzugvolumens bestimmt werden. Auf Grundlage dieser Auswertungsergebnisse kann die Zeitdauer der Atemzugsphasen das tatsächliche Volumen des Atemzugs und das Atemmuster bestimmt werden.

Durch eine statistische Analyse der Eigenschaften mehrerer abfolgender Atemzüge, kann der physiologische Zustand der untersuchten Person weiter bestimmt werden. Auf Grundlage der Extraktion von Merkmalen für jeden einzelnen Atemzug kann eine Rohdatenreduzierung erreicht werden. Aus der statistischen Auswertung der Eigenschaften mehrerer abfolgender Atemzüge kann zwischen obstruktionsrelevantem Schnarchen und obstruktionsunrelevantem Schnarchen unterschieden werden. Eine Typisierung von Oszillationseigenschaften in Verbindung mit Schnarchereignissen kann hierbei unter Verzicht auf eine Mikrophoneinrichtung erfolgen.

Das Auftreten etwaiger schnarchbedingter Oszillationen kann anhand des zeitlichen Verlaufs des Atemsignals erfasst werden. So ist es beispielsweise möglich, durch Schnarchen verursachte Druckoszillationen aus, durch entsprechende Drucksensoreinrichtungen generierten Signalen zu extrahieren. Insbesondere auf Grundlage einer Frequenz- und Amplitudenanalyse z.B. Fast Fourier-Analyse ist es möglich, Schnarchereignisse hinsichtlich ihres Entstehungsortes (Gaumensegel, Kehlkopf...) zu klassifizieren.

Auf Grundlage der verknüpfenden Betrachtung der Auswertungsmerkmale können insbesondere die folgenden obstruktiven Atmungsstörungen (OSA) erkannt werden:
Apnoe, Hypopnoe, flusslimitierte Atmung, sowie stabile und instabile Atmung.

Eine Atmungsstörung wird als Apnoeereignis klassifiziert, wenn ein Atmungsstillstand erfasst wird, dessen Länge eine vorbestimmte Zeitdauer von beispielsweise 10 Sekunden übersteigt.

Ein Hypopnoeereignis kann dann als vorliegend angesehen werden, wenn nach drei beispielsweise als normal klassifizierten Atemzügen, mindestens zwei sowie maximal drei größere Atemzüge erkannt werden. Als weiteres Kriterium hierfür kann das inspiratorisches Differenzvolumen der betrachteten Atemzüge herangezogen werden.

In dem jeweils untersuchten Atemzug kann eine Flusslimitation erkannt werden, wenn der Atemgasstrom während der Inspirationsphase bestimmte Plateauzonen oder mehrere Maxima aufweist.

Etwaige in einem Drucksignal auftretende hochfrequente Oszillationen können in Verbindung mit dem Atemflusssignal als in- oder expiratorisches Schnarchen klasssifiziert werden. Die hinsichtlich des Auftretens von Schnarchen generierten Auswertungsmerkmale können in die zur Generierung der Auswertungsresultate vorgesehene verknüpfende Betrachtung Eingang finden.

Die erfindungsgemäße Erfassung und Auswertung der hinsichtlich des Atemgasstromes indikativen Signale kann Informationen zur Beschreibung und Visualisierung des physiologischen Zustands einer Person insbesondere im Hinblick auf eine mit schlafbezogenen Atmungsstörungen in Verbindung stehende Erkrankung geben. Die erfindungsgemäße Signalerfassung und Auswertung kann zur Konfiguration von Beatmungsgeräten herangezogen werden.

Gemäß einem besonderen Aspekt der Erfindung werden wenigstens zwei der nachfolgenden Angaben kombiniert angewendet:
- Der Grad der statistischen Sicherheit der ermittelten Beurteilungs- oder Klassifizierungsergebnisse wird ermittelt.
- Für jeden Atemzug werden atemzugsspezifische Merkmale nach Maßgabe definierter Analyseprozeduren ermittelt.
- Die Analyseprozeduren berücksichtigen insbesondere den Inspirationsvorgang, den Expirationsvorgang, den Übergang zwischen den genannten Vorgängen, Kurveneigenschaften des Atemgasstromverlaufs innerhalb jedes Atemzyklus, Kombinationsbetrachtungen von Merkmalen des Atemgasstromverlaufes innerhalb eines Atemzuges.

- Die Gemeinsamkeiten von Atemzügen werden ermittelt.
- Unterschiede oder zeitliche Änderungen von Atemzugsmerkmalen werden ermittelt und bei der Beurteilung des physiologischen Zustands des Anwender berücksichtigt.
- Aus einer mehrfach verknüpften Betrachtung von Einzelmerkmalen werden Auswertungsresultate generiert die standardisiert parametrisch einen physiologischen Zustand oder physiologische Eigenschaften beschreiben.

Weitere Einzelheiten und Merkmale der Erfindung ergeben sich aus der nachfolgenden Beschreibung in Verbindung mit der Zeichnung. Es zeigen:
- **Fig.1a**: eine perspektivische Ansicht einer ersten Ausführungsform eines erfindungsgemäßen Erfassungsgerätes;
- **Fig.1b**: eine Skizze zur Illustration einer Nasalkanüleneinrichtung zur Erfassung des Atemgasstromes durch Staudruckabgriff im Bereich der Nasenöffnungen;
- **Fig.1c**: eine Skizze zur Illustration einer Kanüleneinrichtung zur Erfassung des Atemgasstromes durch Druckabgriff im Innenbereich einer Einwegfiltermaske;
- **Fig.1d**: eine Skizze zur Illustration einer Applikationsweise des Erfassungsgerätes am Anwender unter Verwendung eines den Thorax umgreifenden Bandes;
- **Fig.1e**: eine Skizze zur Illustration einer zweiten Ausführungsform eines erfindungsgemäßen Erfassungsgerätes mit mehreren Anschlussabschnitten zum Anschluss von Druckmesskanülen zur separaten Erfassung nasaler und oraler Atmung;
- **Fig.1f**: eine Skizze zur Erläuterung eines Konzeptes zur Überwachung der Atmung durch Abgriff von, für den Atemgasstrom durch die linke Nasenöffnung, die rechte Nasenöffnung sowie den Mund des Anwenders indikativen Signalen
- **Fig.1g**: eine Skizze zur Erläuterung Nasenbrillenanordnung mit integrierter Kanüle zur Erfassung der Mundatmung;
- **Fig.1h**: eine Skizze zur Erläuterung eines Konzeptes zur Überwachung der Atmung durch Abgriff unter Verwendung einer Vollgesichtsmaske;
- **Fig.1i**: eine Skizze zur Erläuterung einer zur Applikation auf der Nase eines Anwenders vorgesehenen Abgriffseinrichtung;
- **Fig.1j**: eine Skizze zur Erläuterung einer für eine Abgriffseinrichtung nach Figur 1i geeigneten Strömungsweggestaltung;
- **Fig.1k**: eine Skizze zur Erläuterung einer zur nasalen Applikation vorgesehenen, die Nasenöffnungen eines Anwenders abdeckenden Abgriffseinrichtung;
- **Fig.1l**: eine Skizze zur Erläuterung einer zum Ansatz an den Nasenöffnungsbereich vorgesehenen Abgriffseinrichtung;
- **Fig.1m**: eine Skizze zur Erläuterung einer weiteren zum Ansatz an den Nasenöffnungsbereich vorgesehenen Abgriffseinrichtung mit Blenden- oder Drosselfenstern;
- **Fig.1n**: eine Skizze zur Erläuterung eines Abgriffkonzeptes mit aktiver Spülluftzufuhr;
- **Fig. 2**: eine Skizze zur Illustration eines modular aufgebauten Erfassungsgerätes, mit einem Game-Boy kompatiblen Untermodul;
- **Fig. 3**: eine Bildschirmansicht zur Illustration einer Dartsellungsmöglichkeit der erfindugsgemäß erhobenen Messdaten unter Verwendung eines Auswertungsgerätes in Forme eines Rechners, z.B. Notebooks oder auch Game-Boys;
- **Fig**. **4a**: ein Diagramm zur Erläuterung des Atemgasstromes für einen einzelnen Atemzug;
- **Fig**. **4b**: ein Diagramm das den zeitlichen Verlauf des Atemgasstromes für mehrere Atemzüge beschreibt;
- **Fig. 4c**: ein Diagramm das den zeitlichen Verlauf des Atemgasdruckes mit einzelnen, durch Schnarchen verursachten Druckoszillationen darstellt;
- **Fig. 4d**: ein Diagramm das den zeitlichen Verlauf des Atemgasstromes für mehrere, durch eine Apnoe-Periode unterbrochene Atemzüge darstellt;
- **Fig. 5**: ein Diagramm das den zeitlichen Verlauf des Atemgasstromes mit einem Hypopnoeereignis darstellt;
- **Fig. 6**: ein Diagramm zum zeitlichen Verlauf des Atemgasstromes für mehrere, zum Teil flusslimitierte Atemzüge;
- **Fig. 7**: ein Diagramm zur Erläuterung des zeitlichen Verlaufs des Atemgasstromes im Falle einer im wesentlichen ungestörten, stabilen Atmung;
- **Fig. 8**: ein Diagramm zur Erläuterung des zeitlichen Verlaufes des Atemgasstromes im Falle einer instabilen, gestörten Atmung;
- **Fig. 9**: ein Diagramm das den zeitlichen Verlauf des Atemgasstromes darstellt wobei in dem Drucksignaloszillationen auftreten die durch Schnarchen verursacht sind;

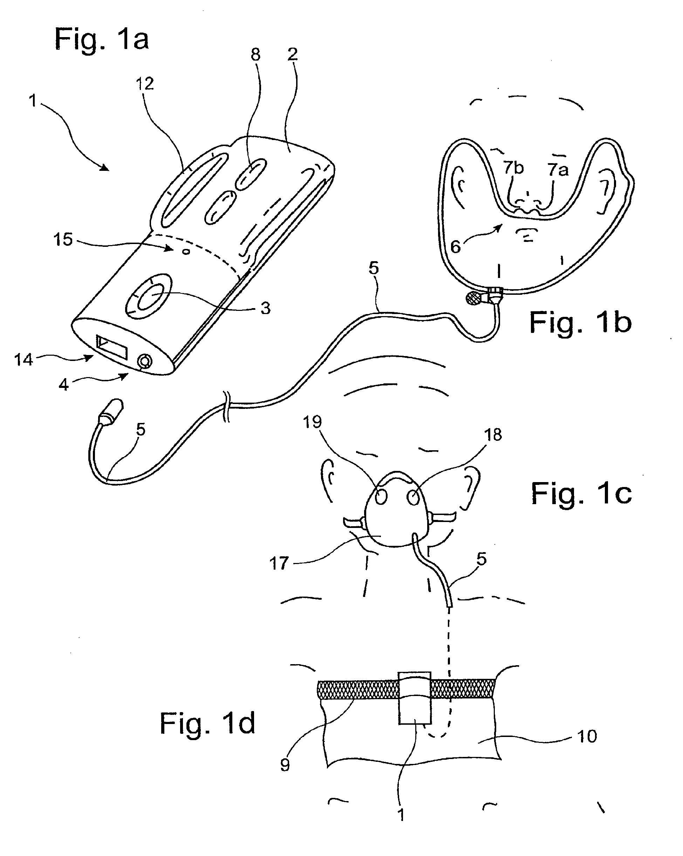

In Figur 1a ist ein erfindungsgemäßes mobiles Erfassungsgerät 1 dargestellt, das eine Gehäuseeinrichtung 2 und eine darin aufgenommene, hier nicht näher dargestellte Druck Sensoreinrichtung zur Erfassung und Generierung eines hinsichtlich des Atemgasstromes indikativen Atemstromsignals aufweist. Das Erfassungsgerät 1 umfasst ferner eine unter Einschluss einer Speichereinrichtung gebildete elektronischen Datenverarbeitungseinrichtung zur Verarbeitung des hinsichtlich des zeitlichen Verlaufs des Atemstrom indikativen Atemstromsignals. Die Datenverarbeitungseinrichtung ist derart konfiguriert, dass diese eine Speicherung von hinsichtlich des zeitlichen Verlaufs des Atemstromsignals indikativen Daten veranlasst.

Das gezeigte Erfassungsgerät 1 eignet sich insbesondere zur Anwendung im häuslichen Bereich, d.h. in der gewohnten Umgebung des Betroffenen zur Aufzeichnung von hinsichtlich der Atmung während einer Schlafphase charakteristischen Merkmale in einer Weise, die es gestattet, den physiologischen Zustand des Betroffenen mit hinreichend hoher Aussagekräftigkeit standardisiert zu bewerten. Auf Grundlage einer standardisierten Auswertung der erfassten Messergebnisse kann beurteilt werden, ob - und ggf. mit welchem Schweregrad das Krankheitsbild OSA vorliegt und ob eine tiefergehende Untersuchung durch ein Schlaflabor zu empfehlen ist.

Die Datenverarbeitungseinrichtung, ist derart konfiguriert, dass eine Prüfung erfolgt, ob das erfasste Atemstromsignal vorgegebene Signalqualitätskriterien erfüllt. Für den Fall, dass das anliegende Signal bestimmte Kriterien nicht erfüllt, wird die Signalaufzeichnung unterdrückt, und ein Dateieintrag veranlasst, aus welchem die zeitliche Lage von Perioden mit als unzulässig klassifizierten Signalen hervorgeht.

Die Datenverarbeitungseinrichtung ist derart ausgebildet, dass diese Zugriff zu einer Zeitgebereinrichtung hat, so dass die hinsichtlich des Atemstromsignals indikativen Daten mit einer Zeitinformation verknüpft aufgezeichnet werden können.

In Verbindung mit jener Datenverarbeitungseinrichtung ist ein Datenkompressionssystem verwirklicht, durch welches das erfasste, sich zeitlich ändernde Signal komprimiert aufgezeichnet wird.

Die Datenverarbeitungseinrichtung ist derart konfiguriert, dass der Aufzeichnungsvorgang durch einen anwenderseitig veranlassten Schaltimpuls ausgelöst wird. Die Einschaltung erfolgt indem der Schalttaster 3 über eine vorgegebene Mindestdauer von z.B. 3 Sekunden betätigt wird.

Die Datenverarbeitungseinrichtung ist derart ausgebildet, dass diese den Aufzeichnungsbeginn bzw. die Datenspeicherung veranlasst, wenn die anliegenden Atemstromsignale ein bestimmtes Kriterium z.B. ein vorgegebenes Periodizitätskriterium erfüllen.

Das Erfassungsgerät 1 weist einen ersten Druckmessanschluss 4 auf, an welchen eine Messkanüle 5 anschließbar ist.

Wie in Figur 1b dargestellt ist, kann diese Messkanüle 5 mit einer Nasaldruckmessbrilleneinrichtung 6 verbunden sein, zum Abgriff eines Staudruckereignisses aus dem, aus den Nasenöffnungen 7a, 7b abströmenden Atemgasstrom.

Es ist möglich, das Erfassungsgerät 1 mit einem zweiten Druckmessanschluss auszustatten, zur Erfassung eines zweiten Druckmesssignals. Durch die Möglichkeit der Erfassung von zwei Druckmesssignalen wird es möglich, das Erfassungsgerät 1 derart zu betreiben, dass dieses für die linke und rechte Nasenöffnung 7a, 7b jeweils indikative Atemstromsignale erfassen kann.

In Figur 1c ist eine weitere Variante zur Generierung eines hinsichtlich des Atemgasstromes indikativen Signals dargestellt. Diese Variante umfasst eine in der Art eines Mundschutzes ausgebildete Maske 17 aus einem gasdurchlässigen Material (z.B. Einweg-Papierfiltermatrerial). Durch diese Maske 17 wird es möglich, im Zusammenhang mit der Atmung im unmittelbaren Umgebungsbereich der Mund- und Nasenöffnung eine Druckdifferenz gegenüber der Umgebung zu erzeugen. Diese Druckdifferenz wird insbesondere durch die Dichtigkeit des Mundschutzes sowie die Durchlässigkeit des Masken- oder Filtermaterials bestimmt. Etwaige Nichtlinearitäten können im Bereich des Erfassungsgerätes ermittelt und kompensiert werden.

Der durch den als Diaphragma wirksamen Mundschutz definierte Maskeninnenraum steht über die Messkanüle 5 mit dem Erfassungsgerät in Verbindung. Es ist möglich, an der Maske Klappen- oder Ventileinrichtungen 18, 19 vorzusehen, durch welche der Einatmungsvorgang erleichtert wird. Über diese Ventil- oder Klappeneinrichtungen, insbesondere den Öffnungsgrad derselben ist es ebenfalls möglich, hinsichtlich der Atmung indikative Signale zu erfassen. Diese Signalübertragung kann drahtlos, insbesondere optisch, beispielsweise durch Infrarotlicht erfolgen.

Das hinsichtlich des Atemgasstromes indikative Signal kann weiterhin auch über anderweitige Messmittel, insbesondere Messblenden oder Volumenstromsensoren erfasst werden.

Das Erfassungsgerät 1 kann eine Einrichtung 8 zur Erfassung der Thoraxweitung aufweisen. Die Einrichtung zur Erfassung der Thoraxweitung kann wie in Figur 1d dargestellt ist ein Bandelement 9 umfassen, das um den Thoraxbereich des Anwenders 10 herumführbar ist. Es ist möglich, dieses Bandelement 9 derart auszugestalten, dass aus diesem ein hinsichtlich der Banddehnung, oder Bandbelastung indikatives Signal ableitbar ist. Es ist auch möglich, an dem Erfassungsgerät 1 Vorkehrungen zu treffen, durch welche die Bandbelastung erfasst werden kann. Insbesondere ist es möglich, an dem Erfassungsgerät 1 eine Schlaufeneinrichtung 12 vorzusehen, durch welche die Bandweitung, oder Bandkräfte erfasst werden können. Es ist auch möglich, an dem Erfassungsgerät 1 Druck- oder Krafterfassungsstrukturen 8 vorzusehen, durch welche die durch ein über diese Krafterfassungsstrukturen geführtes Band aufgebrachten Kräfte erfasst werden können. Das Band 9 zur Erfassung der Thoraxweitung kann zugleich der Befestigung des Erfassungsgeräts 1 am Anwender dienen. Es ist auch möglich, das Thoraxband lediglich zur Befestigung des Erfassungsgerätes 1 zu verwenden und von einer Erfassung des der Thoraxweitung abzusehen.

An dem Erfassungsgerät 1 können Elektrodeneinrichtungen vorgesehen sein durch welche EKG Signale vom Anwender abgegriffen werden können, indem das Erfassungsgerät unmittelbar auf die Haut des Anwenders aufgesetzt wird. Diese EKG-Signale können ebenfalls in zeitlicher Zuordnung aufgezeichnet werden.

Der Aufzeichnungsvorgang ist über den Schalttaster 3 anwenderseitig veranlassbar beendbar. Es ist weiterhin auch möglich, das Erfassungsgerät 1 derart zu konfigurieren, dass der Aufzeichnungsvorgang bei Erfüllung eines Zeitkriteriums beendet wird. Insbesondere ist es möglich, den Aufzeichnungsvorgang zu beenden, wenn dieser eine vorgegebene Aufzeichnungsdauer von z.B. 9,5 h erreicht hat.

Der Aufzeichnungsvorgang wird ferner auch schaltungsgesteuert beendet, wenn das anliegende Atemstromsignal über ein bestimmtes Abschaltzeitfenster ein bestimmtes Abschaltkriterium erfüllt.

Das Erfassungsgerät 1 ist mit einer Schnittstelleneinrichtung 14 versehen, zur Übertragung der aufgezeichneten Daten auf ein externes Auswertungssystem. Diese Schnittstelleneinrichtung ist hier als USB-Schnittstelle ausgeführt. Das Erfassungsgerät umfasst auch eine Infrarot-Schnittstelle zum potentialfreien Signalabgriff. Über diese Infrarotschnittstelle können während des Betriebs des Geräts die gewonnenen, hinsichtlich der Atmung generierten Signale zur anderweitigen Verwertung ausgelesen werden. Das Erfassungsgerät 1 kann insoweit als Messumformer betrieben werden.

Es ist auch möglich, das Erfassungsgerät 1 derart zu gestalten, dass die Speichereinrichtung austauschbar in dem Erfassungsgerät 1 aufgenommen, oder an diesem anbringbar ist. Die Speichereinrichtung kann hierzu als Karte oder insbesondere USB-Flashstick ausgebildet sein. Es ist möglich, durch einen vorab auf der Speichereinrichtung veranlassten Eintrag personenspezifische Daten auf dem Speichermedium aufzuzeichnen. Auf Grundlage dieser Vorab-Aufzeichnungen ist es möglich, eine Vorkonfiguration des Erfassungsgerätes 1 vorzunehmen, oder eine zutreffende Zuordnung der erfasten Daten zu dem Anwender sicherzustellen.

Die Konfiguration der Datenverarbeitungseinrichtung ist durch ein Datenverarbeitungsprogramm festgelegt, wobei dieses Datenverarbeitungsprogramm vorzugsweise veränderbar oder wechselbar ist. Die Abbildung des Datenverarbeitungsprogramms im Bereich des Erfassungsgerätes 1 kann über eine ROM-Einrichtung oder eine RAM-Einrichtung, insbesondere über die vorgenannten Schnittstelleneinrichtungen 14, zusätzliche Schnittstelleneinrichtungen, oder auch das Speichermedium erfolgen.

An dem Erfassungsgerät 1 sind neben einer vorzugsweise intuitiv leicht zu bedienenden Schaltereinrichtung 3 auch Anzeigeeinrichtungen 15 vorgesehen, zur Anzeige der Betriebsbereitschaft oder des Funktionszustands des Erfassungsgerätes. Bei der gezeigten Ausführungsform erfolgt die Anzeige der Aufzeichnungsbereitschaft des Erfassungsgerätes durch periodisches Aufblinken einer grünen Signaldiode.

Das Erfassungsgerät 1 umfasst eine Spannungsversorgungseinrichtung, die hier durch eine Batterieeinrichtung gebildet ist. Die Batterieeinrichtung ist kompakt ausgebildet, so dass das Erfassungsgerät flach und kleinbauend ausgeführt werden kann und nur ein geringes Eigengewicht aufweist.

Die Datenverarbeitungseinrichtung ist mit einer Kalibriereinrichtung gekoppelt, zur Kalibrierung des Atemstromsignals. Die Kalibriereinrichtung ist derart ausgebildet, dass diese eine selbsttätige Anpassung des Systems an die erfassten Signalpegel vornimmt.

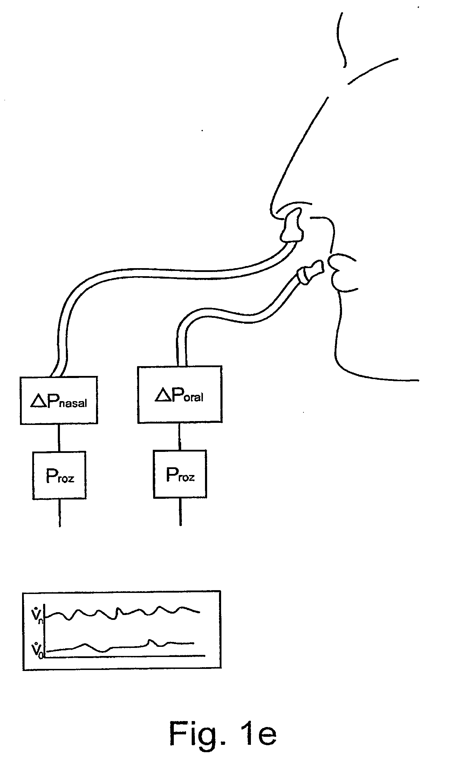

Figur 1e zeigt eine Skizze zur Illustration einer zweiten Ausführungsform eines erfindungsgemäßen Konzeptes zum separaten Abgriff eines hinsichtlich der Nasalatmung und eines hinsichtlich der oralen Atmung indikativen Signals. Diese Signale können als Drucksignale über Messkanülen erfasst werden. Die Signale können insbesondere als eine Druckdifferenz gegenüber dem Umgebungsdruck angebende Druckdifferenzsignale, gewonnen werden, die Signale können durch Signalverarbeitungsprozeduren normiert und überarbeitet werden. Die überarbeiteten Signale können zur Beschreibung, insbesondere Visualisierung des zeitlichen Verlaufs der Atmung verwendet werden.

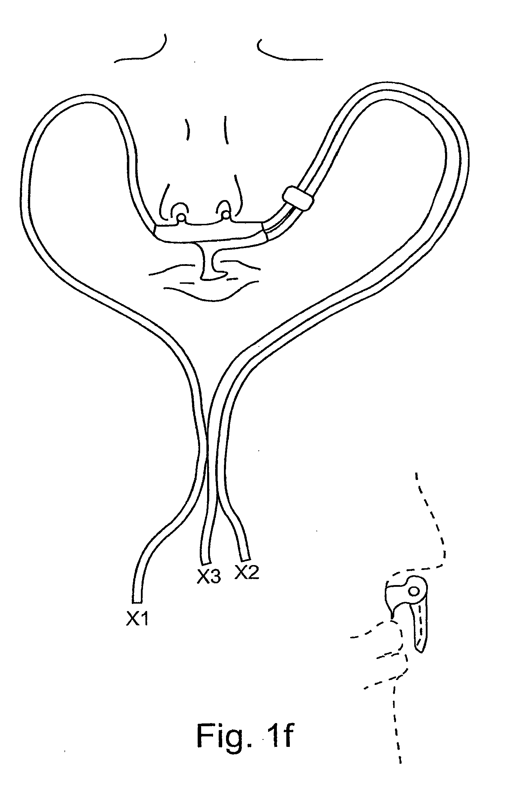

Fig.1f zeigt eine Skizze zur Erläuterung eines Konzeptes zur Überwachung der Atmung durch Abgriff von, für den Atemgasstrom durch die linke Nasenöffnung, die rechte Nasenöffnung sowie den Mund des Anwenders indikativen Signalen X1, X2, X3. Diese Signale können insbesondere über Druckmesskanülen abgegriffen werden. Die Signale X1, X2 können beispielsweise als Staudrucksignale durch eine Nasenbrillenanordnung erhoben werden. Das Signal X3 kann durch eine Messkanüle erfasst werden, die im Bereich der Oberlippe des Anwenders in einen Strömungsbereich eintaucht, über welchen ein Gasaustausch bei Mundatmung erfolgt. Es ist möglich, die Signale X1, X2, X3 zusammengefasst derart auszuwerten, dass das Summensignal ein Plausibilitätskriterium, beispielsweise im Hinblick auf das Atemzugsvolumen erfüllt.

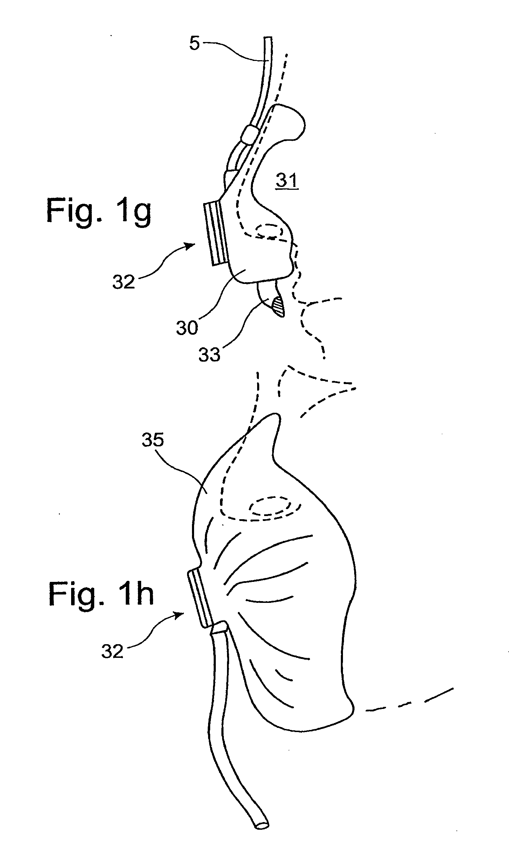

Fig.1g zeigt eine Skizze zur Erläuterung einer nasal zu applizierenden Abgriffseinrichtung mit integrierter Einrichtung zur Erfassung der Mundatmung. Die Abgriffseinrichtung umfasst einen Basiskörper 30, der aus einem Elastomermaterial, insbesondere Silikonkautschuk gefertigt ist. Der basiskörper definiert einen den Nasenspitzenbereich 31 eines Anwenders aufnehmenden und die Nasenöffnungen übergreifenden Abgriffsinnenraum. Dieser Abgriffsinnenraum steht über eine Messblendeneinrichtung 32 mit der Umgebung in Verbindung. Die Messblendeneinrichtung ist derart ausgebildet, dass diese im rahmen der Atemgasverlagerung zwischen dem Abgriffsinnenraum und der Umgebung einen relativ geringen, jedoch definierten Strömungswiderstand verursacht. Die infolge des Strömungswiderstandes der Messblendeneinrichtung im Abgriffsinnenraum gegenüber der Umgebung auftretenden Druckänderungen können über die Messkanüle 5 erfasst und zur Gewinnung hinsichtlich der nasalen Atmung indikative Daten konvertiert werden.

An dem Basiskörper 30 ist eine Sensoreinrichtung 33 vorgesehen, zum Abgriff eines hinsichtlich der Mundatmung indikativen Ereignisses, insbesondere Druckschwankung. Diese Druckschwankung kann ebenfalls durch eine Messkanüle oder anderweitige Signalübertragungseinrichtung einer weiteren Aufzeichnung zugeführt werden. Durch die Applikation einer zur Erfassung der oralen Atmung vorgesehenen Messeinrichtung über eine auf der Nase des Anwenders aufsitzende Struktur wird eine besonders vorteilhafte, insbesondere lagestabile und reproduzierbare Anordnung dieser Messeinrichtung gewährleistet.

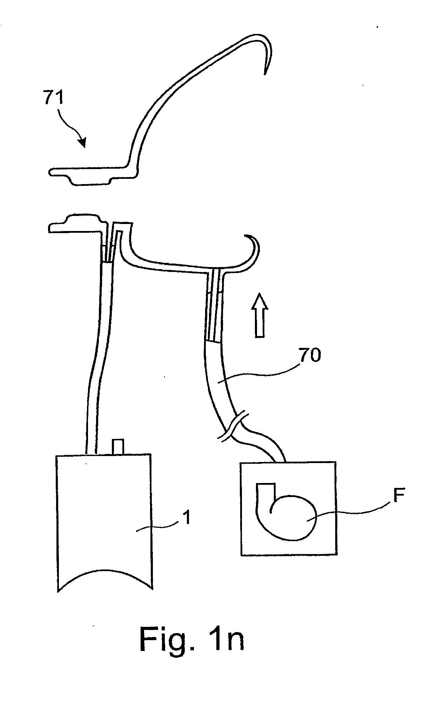

Die Messblende 32 kann als Gitter, Sieb, oder auch Gewebeelement ausgeführt sein. Wie später noch in Verbindung mit Figur 1n ausgeführt wird, ist es möglich, den Abgriffsinnenraum durch aktive Zufuhr eines atembaren Gases, insbesondere Umgebungsluf zu spülen. Hierdurch wird es möglich, auch bei besonders flacher Atmung einen ausreichenden Luftaustausch sicherzustellen. Weiterhin wird es möglich, das über die Kanüle 5 abgreifbare Drucksignal bei flacher Atmung noch in den positiven Bereich zu versetzen. Ein Unterdruck tritt dann erst auf, wenn der Inspirationsstrom größer ist als der Spülstrom.

Figur 1h zeigt eine Skizze zum Abgriff eines nasale und orale Atmungg zusammenfassenden Signals unter Verwendung einer die Nase und den Mundbereich übergreifenden Maskeneinrichtung 35. Die Maskeneinrichtung kann aus einem luftdurchlässigen Material gefertigt sein, oder wie hier gezeigt mit einer Drossel- oder Messblendeneinrichtung 32 versehen sein.

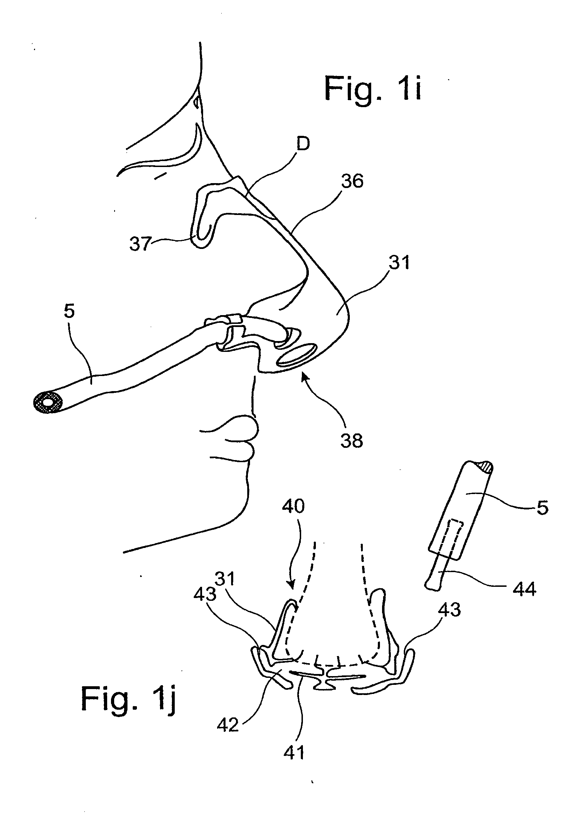

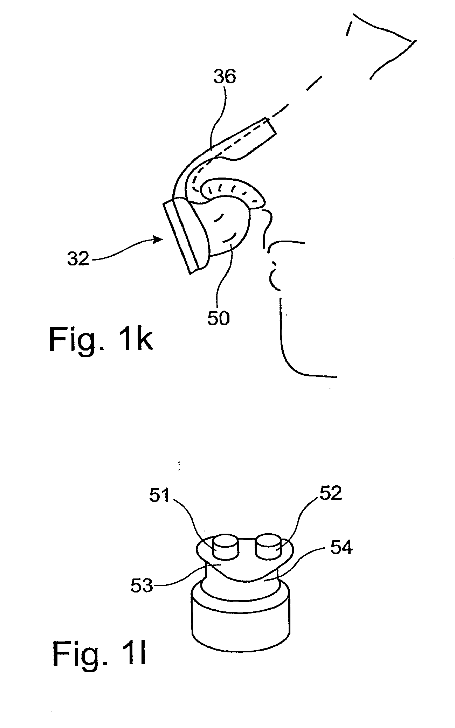

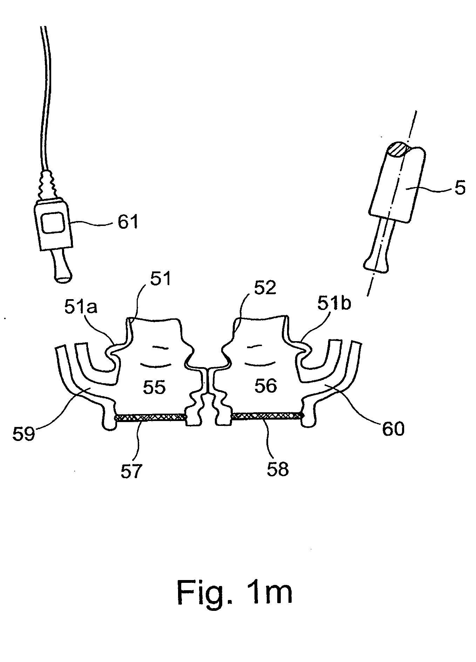

Fig.1i veranschaulicht eine Abgriffseinrichtung zum Abgriff eines hinsichtlich der nasalen Atmung indikativen Signales. Ähnlich wie die Variante nach Figur 1 umfasstt diese Abgriffseinrichtung einen Basiskörper 30 der einen die Nasenspitze aufnehmenden Abgriffsinnenraum definiert. Der Basiskörper 30 ist aus einem Kunststoffmaterial, vorzugsweise einem transparenten Elastomermaterial gefertigt.

Der Basiskörper 30 umfasst einen auf dem Nasenrücken verlaufenden Haltesteg 36. An dem Haltesteg 36 sind Halteflügel 37 ausgebildet. Der Haltesteg 36 und die Halteflügel 37 können über Klebebänder am Anwender fixiert werden, oder ggf, abschnittsweise selbstklebend ausgebildet sein. In dem Haltesteg 36 und/oder den Halteflügeln 37 können biegsame Einlagen, insbesondere Drahtabschnitte D verlaufen durch welche die Abgriffseinrichtung an die individuelle Nasentektur des Anwenders anpassbar ist.

Der Basiskörper 31 definiert einen Luftaustrittsabschnitt 38 über welchen eine Atemgasverlagerung von/zu den nasalen Atemwegen und der Umgebung erfolgen kann. Der Luftaustrittsabschnitt 38 kann so ausgebildet sein, dass dieser einen definierten Strömungswiderstand verursacht, so dass ein im Bereich des Abgriffsinnenraums herrschender Druck als hinsichtlich der Atmung indikatives Signal z.B. über die hier gezeigte Kanüle 5 abgegriffen werden kann.

Der Basiskörper 31 kann insbesondere in seinem, in Applikationsposition den Nasenöffnungen benachbartem Bereich so gestaltet sein, dass eine besonders vorteilhafte Erfassung der nasalen Atemgasströmung ermöglicht wird. Ein hierzu besonders geeigneter Aufbau ist Figur 1j skizziert.

Der Basiskörper 31 muss nicht zwingend aus einem Kunststoffmaterial gefertigt sein. Es ist auch möglich diesen aus einem Papier-, Zellstoff, Faser- oder anderweitigen insbesondere für Einweggebrauch geeigneten Material zu fertigen. Im Innenbereich des Basiskörpers kann abschnittsweise ein Schaumstoff- oder anderweitiges Polstermaterial vorgesehen sein, durch welches eine Abdichtung und Polsterung, insbesondere im Nasenrückenbereich erreicht wird.

Der in Figur 1j im Querschnitt vereinfacht dargestellte Basiskörper 31 ist mit einer Dichtlippenstruktur 40 versehen welche einen Abgriffsinnenbereich gegenüber der Umgebung abdichtet.

Der Abgriffsinnenbereich enthält eine Luftführungsstruktur die in dem die Nasenöffnungen umgebenden Bereich auf der Nase des Anwenders aufsitzt. Bei diesem Ausführungsbeispiel ist die Luftführungsstruktur so ausgebildet, dass diese eine hinsichtlich der linken und der rechten Nasalströmung separate Signalerhebung ermöglicht.

Die Luftführungsstruktur umfasst eine Umlenkplatte 41 über welche die über die Nasenöffnungen strömende Luft umgelenkt wird. In dem so geschaffenen Umlenkpfad ist an einer typischen Staudruckstelle ein Druckabgriffsport 42 vorgesehen. Der in diesem Druckabgriffsport herrschende Druck kann über eine Messkanüle 5 jeweils abgegriffen werden.

Der hier im Querschnitt gezeigte Basiskörper 31 ist aus einem Elastomermaterial gefertigt. Im Bereich des jeweiligen Druckabgriffsport sind elastische Einsteckkanäle 43 vorgesehen, in welche ein Einsteckstutzen 44 zur Ankoppelung der jeweiligen Kanüle 5 einsteckbar ist.

Der Umlenkpfad ist so ausgebildet, dass durch diesen eine Luftstromumlenkung um etwa 180° erfolgt. Der Druckabgriffsport 42 befindet sich jeweils im Bereich der Umlenkstelle.

Die Umlenkplatte 41 kann so ausgebildet sein, dass diese elastisch nachgiebig ist und bei höheren Atemgasströmen größere Durchgangsquerschnitte freigibt, es ist auch möglich, die Atemgasströmung anhand der Auslenkung der Umlenkplatte zu erfassen. Um ein Verstopfen des Druckabgriffports 42 zu vermeiden ist es möglich, die Messkanüle 5 permanent oder intermittierend mit einem Spülluftstrom zu beaufschlagen.

Figur 1k zeigt eine Abgriffseinrichtung mit jeweils in die Nasenöffnungen eingeführten und über einen auf dem Nasenrücken sitzenden Steg 36 fixierten Abgriffselementen 50. Die Abgriffselemente 50 sind aus einem elastomeren Material gefertigt und derart konturiert, dass diese vorteilhaft an den Nasenöffnungsbereich eines Anwenders ansetzbar sind. Die Abgriffselemente bilden Messkanalabschnitte über welche eine Erfassung des nasalen Atemgasstromes ermöglicht wird. Es ist möglich, diese Messkanalabschnitte an eine Messblende- oder Drosseleinrichtung 32 heranzuführen, über welche ein definierter Strömungswiderstand oder eine hinsichtlich der Signalerhebung vorteilhafte Strömungsbeeinflussung ermöglicht wird.

Figur 1l zeigt ein Abgriffselement mit zwei in die jeweilige Nasenöffnung einsetzbaren elastischen Stutzenabschnitten 51, 52, sowie einen eine axiale Abstützung ermöglichenden Bodenabschnitt 53. Beide Stutzenabschnitte 51, 52 münden bei diesem Ausführungsbeispiel in einen gemeinsamen Messkanalabschnitt 54 der über eine Messblende in die Umgebung mündet.

In Figur 1m ist in Form einer vereinfachten Querschnittsskizze eine weitere Variante einer Vorrichtung zum Abgriff hinsichtlich der nasalen Atemgasströmung indikativer Signale dargestellt. Bei diesem Ausführungsbeispiel sind die Stutzenabschnitte 51, 52 mit Balgstrukturen 51 a, 51 b versehen.

Die Abgriffseinrichtung bildet zwei Messkanäle 55, 56 die über Blenden- oder Drosselelemente 57, 58 mit der Umgebung in Verbindung stehen. Die Drosselelemente 57, 58 sind in eine Umfangsnut eingesetzt, die Mündungsbereich des jeweiligen Messkanals 55, 56 ausgebildet ist.

Der Abgriff des jeweiligen Drucksignals aus dem Bereich der Messkanäle erfolgt über Druckabgriffsportabschnitte 59, 60.

Die Signalerhebung kann wie vorangehend beschrieben über eine Kanüle 5 oder einen unmittelbar anschließbaren oder einsteckbaren Messumformer 61 erfolgen. Ober den Messumformer 61 kann das hinsichtlich des Druckes in dem jeweiligen Messkanal indikative Signal in ein elektrisches, oder auch optisches Signal umgewandelt werden.

In Figur 1n ist skizzenhaft eine Messanordnung dargestellt, bei welcher der von einer Abgriffseinrichtung definierte Abgriffsinnenraum aktiv mit Spülluft beaufschlagt wird. Hierzu ist eine Spolluftleitung 70 vorgesehen, die in den Abgriffsinnenraum mündet. Die Spülluftzufuhr kann über eine Gebläseeinrichtung oder vorzugsweise über eine statische Fördereinrichtung, z.B. Zahnradpumpe oder anderweitigen volumetrischen Fördereinrichtung F erfolgen.

Die zwischen dem Abgriffsinnenraum und der Umgebung erfolgende Luftverlagerung kann über einen Pneumotachographen 71 erfasst und zur weiteren Auswertung durch das Erfassungsgerät 1 aufgezeichnet werden. Der sich insoweit ergebende Offset des Atemgasflussignales kann über die Auswertungsprozedur berücksichtigt werden. Die Spülluftleitung 70 kann einen kleinen Querschnitt von beispielsweise 10 mm² aufweisen, das Spülvolumen kann sich im Beriech von 1 bis 5 I/min bewegen.

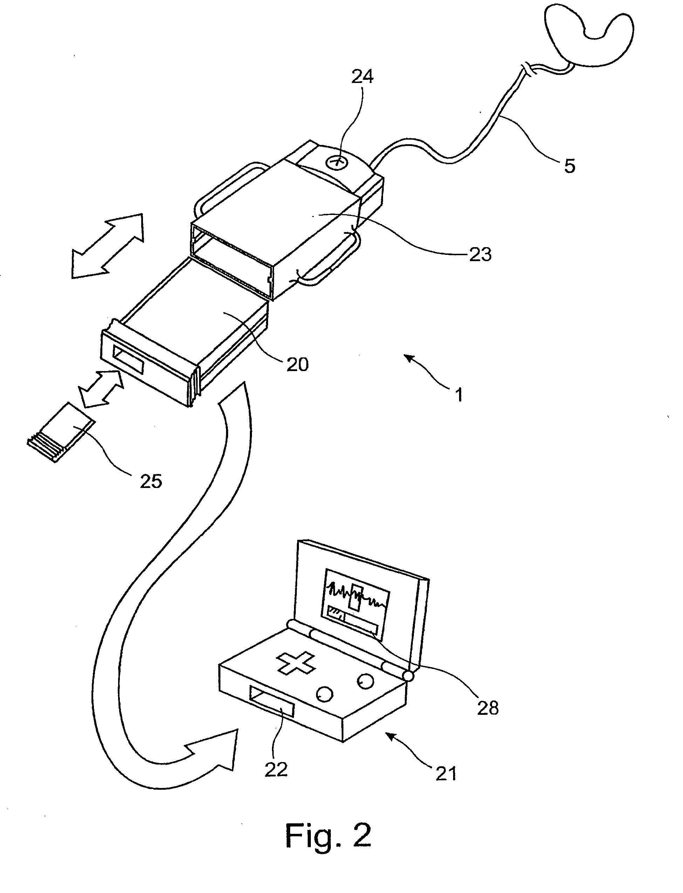

In Figur 2 ist eine Variante des Erfassungsgeräts 1 gezeigt, die eine Strukturkomponente 20 aufweist, die zu einem Wiedergabegerät 21 kompatibel ist, das in seinem Aufbau einem Game-Boy entspricht. Das Erfassungsgerät 1 ist damit derart gestaltet, dass zumindest ein Abschnitt desselben in einen Einschubschacht 22 eines Game-Boy einführbar ist.

Das Erfassungsgerät 1 ist derart ausgebildet, dass dieses ein Basismodul 23 und ein mit diesem koppelbares Aufzeichnungstransfermodul 20 umfasst.

Jenes Aufzeichnungstransfermodul 20 ist als Game-Boy kompatible Struktur ausgebildet. Hierdurch wird es möglich, die aufgezeichneten Daten über eine intuitiv leicht verständliche Benutzeroberfläche über ein handelsübliches Nutzerendgerät 21 zu visualisieren und ggf. im Hinblick auf ausgewählte Eigenschaften auszuwerten und zu verarbeiten. So ist es insbesondere möglich, ein zusammenfassendes Ergebnis in Form eines Schweregradbalkens 28 auszugeben. Durch dieses Balkendiagramm wird verdeutlicht, ob - und in welchem Ausmaß - eine behandlungsrelevante Störung vorliegt, oder nicht

Über das Basismodul 23 erfolgt vorzugsweise die Spannungsversorgung, sowie die Konvertierung des Drucksignales, über eine Kanüle 5 vom Anwender abgreifbar ist. Das Basismodul 23 umfasst hierzu einen Batterieschacht und einen Drucksensor sowie eine Schalteinrichtung 24.

Das Aufzeichnungsmodul 20 umfasst eine Datenverarbeitungseinrichtung die derart konfiguriert ist, dass diese eine Aufzeichnung von hinsichtlich des zeitlichen Verlaufs der Atmung indikativen Signalen auf einer Speichereinrichtung veranlasst. Das Aufzeichnungsmodul 20 kann mit einer Schnittstelleneinrichtung 14 versehen sein, zum Auslesen der aufgezeichneten Daten. Es ist möglich, die Speichereinrichtung 25 lösbar mit dem Aufzeichnungsmodul 20 zu koppeln, so dass es möglich wird, die Speichereinrichtung 25 von dem Aufzeichnungsmodul zu trennen und einem anderweitigen System zur weiteren Auswertung und Visualisierung zuzuführen.

Die Erfassung des Atmungssignals kann alternativ zu einer Erfassung unter Verwendung Nasalkanüleneinrichtung 5 auch über anderweitige Messmittel erfolgen.

Unter Verwendung des vorangehend genannten Erfassungsgerätes 1 wird es möglich ein hinsichtlich des physiologischen Zustands eines Anwenders indikatives Auswertungsresultats auf der Grundlage von Messsignalen die mit der Atmung einer Person im Zusammenhang stehen zu gewinnen, wobei aus den genannten Messsignalen unter Heranziehung standardisierter, Auswertungssysteme, Auswertungsmerkmale generiert werden und im Rahmen eines hierauf basierenden Resultat-Generierungsschrittes wenigstens ein Auswertungsresultat generiert werden kann, das den Schweregrad einer ggf. vorliegenden Erkrankung nach vorgegebenen Auswertungskriterien angibt, insbesondere visualisiert, z.B. in Form eines Balkendiagramms.

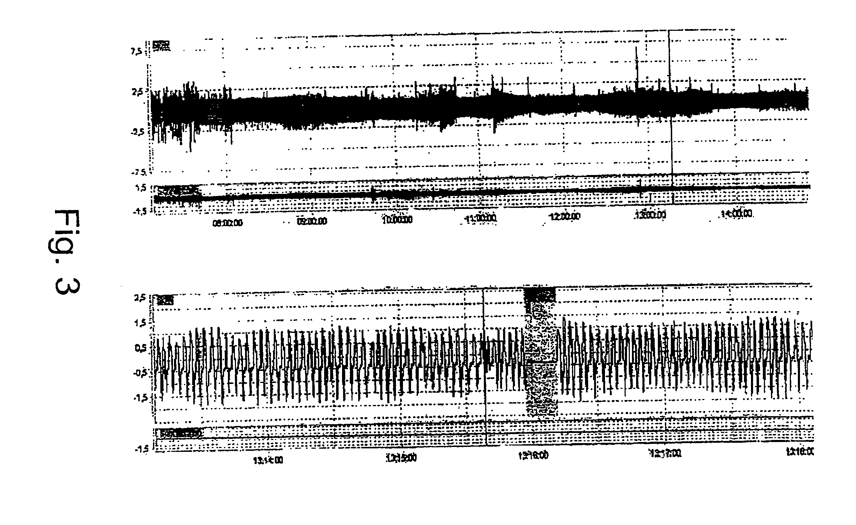

Die gesamten erfassten Daten können weiteren Auswertungsprozeduren zugeführt und wie beispielhaft in Figur 3 dargestellt unter einer komfortablen Menueoberfläche, graphisch visualisiert werden.

Durch das erfindungsgemäße Erfassungsgerät und das mit diesem durchführbare Signalverarbeitungsverfahren wird es auf vorteilhafte Weise möglich, im Rahmen einer ca. 6 bis 8 Stunden andauernden anwenderseitig ambulant durchgeführten Signalerhebung eine Datenmenge zu schaffen, aus welcher Auswertungsmerkmale generiert werden können, aus welchen mit hoher Aussagefähigkeit in einer standardisiert wiederholbaren Weise Auswertungsresultate gewonnen werden können, die in vorteilhafter Weise einer nachfolgenden Diagnose zugrundegelegt werden können und damit eine zu einer standardisierten Bewertung beitragen.

Weitere Einzelheiten insbesondere für die Klassifizierung und automatische Bewertung der Atmung, ergeben sich aus der nachfolgenden Beschreibung.

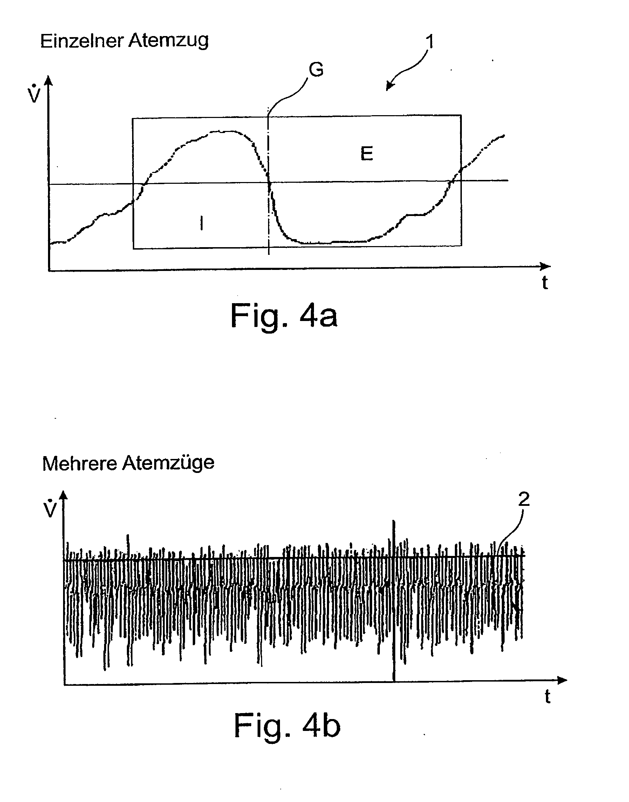

Der in Fig. 4a bezüglich des zeitlichen Verlaufs des Atemgasstromes dargestellte Atemzug 1 umfasst eine Inspirationsphase I und eine Expirationsphase E. Die Ermittlung der Atemphasengrenze G zwischen der Inspirationsphase und der Expirationsphase erfolgt durch überlagerte Auswertung mehrerer Kurvendiskussionskriterien, insbesondere auch unter Berücksichtigung des momentan vorherrschenden Atemmusters sowie der Extremwerte des Atemgasstromes und des Musters, des ermittelten Atemzugsvolumens sowie unter Berücksichtigung der Atemphasenperioden vorangegangener Atemzüge.

Der in Fig. 4a dargestellte Verlauf des Atemgasstromes beschreibt den Atemgasstromverlauf bei einem ungestörten Atemzug. Die Atemzugsbewertung kann anhand der zeitlichen Verhältnissen, z.B. der Inspirations- und Exspirationzeit zueinander oder zu anderen Eigenschaften z.B. der Gesamtatemzugslänge erfolgen. Gemäß einer besonders bevorzugten Ausführungsform der Erfindung wird der Quotient aus der Inspirationszeit und der Gesamtatemzugslänge berechnet um Veränderungen in der Atmung zu erkennen. In Fig. 4b ist der Verlauf des Atemgasstromes über ein längeres Zeitfenster hinweg dargestellt. Wie aus dieser Darstellung erkennbar ist, variieren die einzelnen Atemzüge insbesondere hinsichtlich in der hierbei auftretenden Minimas/Maximas. Die in dieser Darstellung eingetragene Horizontallinie 2 verdeutlicht den statistisch gesehen für Inspirationsphasen mit der höchsten Wahrscheinlichkeit auftretenden maximalen Atemgasstrom. Zusätzlich kann eine statistische Analyse der Inspiration- Exspirations- und Gesamtatemzugszeit über mehrere Atemzüge (vorzugsweise 10 Atemzüge) erfolgen.

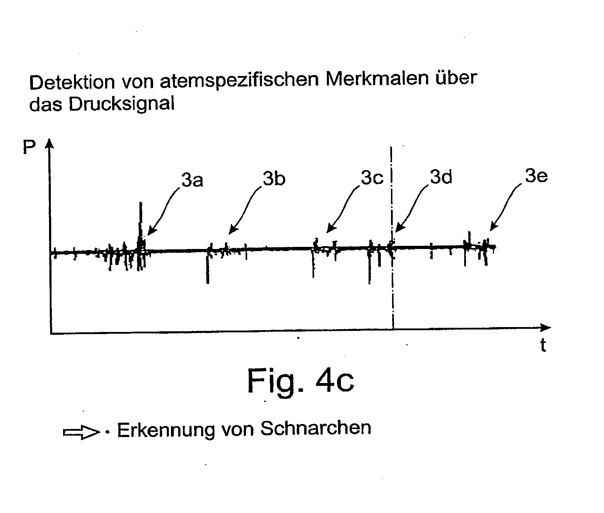

In Fig. 4c ist der zeitliche Verlauf eines hinsichtlich des Atemgasdruckes indikativen Signales dargestellt, wobei dieses Signal Oszillationssequezen 3a, 3b, 3c, 3d und 3e aufweist die durch Schnarchen verursacht sind. Die durch Schnarchen verursachten Druckschwankungen können über eine Anwendernahe Druckerfassungseinrichtung beispielsweise einen Atemgas-Druckmessschlauch erfasst werden. Es ist auch möglich, derartige Druckschwankungen über Mikrophoneinrichtungen zu erfassen.

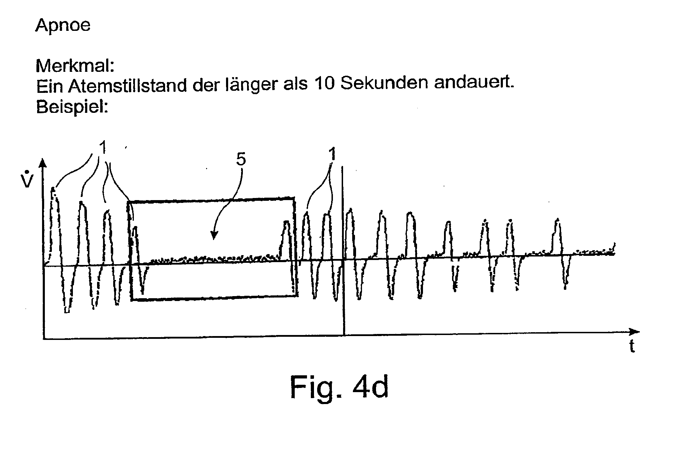

In Fig. 4d ist der zeitliche Verlauf des Atemgasstromes für mehrere, durch eine Atemstillstandsperiode 5 unterbrochene Atemzüge 1 dargestellt. Die auf Grundlage des Atemgasstromes erfasste Atemstillstandsperiode 5 weist eine, einen vorgegebenen Grenzwert von beispielsweise 10 Sekunden überschreitende Zeitdauer auf und wird damit als Apnoe-Phase klassifiziert.. Sowohl die in dieser Darstellung vor der Atemstillstandsperiode 5 erfassten Atemzüge sowie die nachfolgenden Atemzüge zeigen Flusslimitationsmerkmale die in Zuordnung zu jedem Atemzug aufgezeichnet werden.

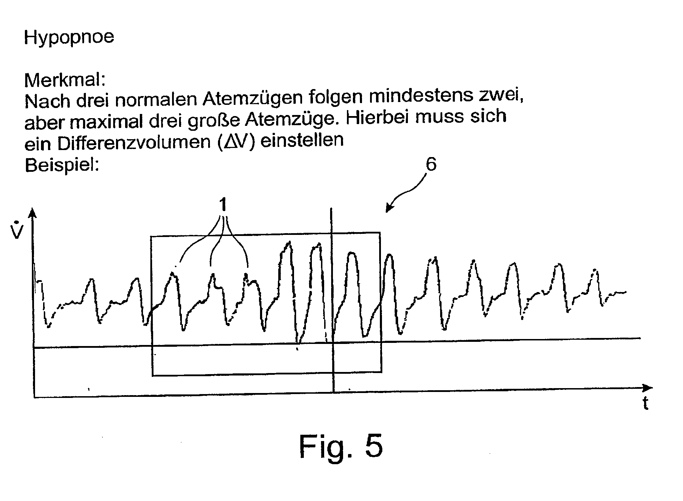

Fig. 5 zeigt den zeitlichen Verlauf des Atemgasstromes mit einer darin enthaltenen Hypopnoephase 6. Die Hypopnoephase 6 wird dann als vorliegend angesehen, wenn nach drei als normal eingestuften Atemzügen 1 mindestens zwei jedoch maximal drei Atemzüge folgen deren Differenzvolumen gegenüber den drei vorangegangenen Atemzügen einen vorgegebenen Grenzwert überschreitet.

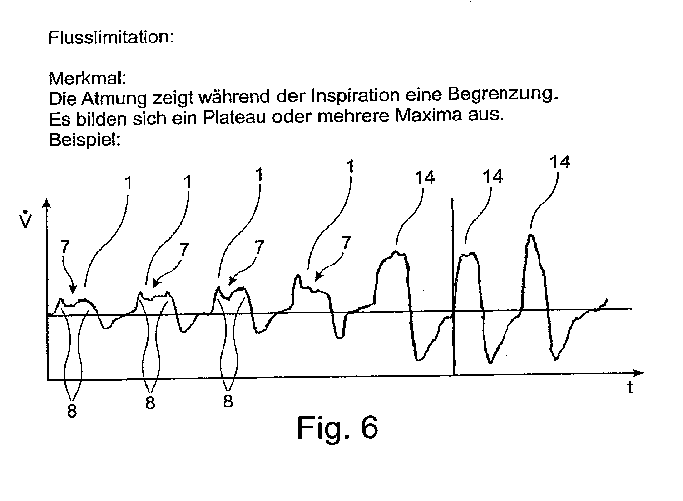

Fig. 6 zeigt den zeitlichen Verlauf des Atemgasstromes für mehrere Atemzüge, wobei die hier sichtbaren ersten 4 Atemzüge 1 Flusslimitationsmerkmale zeigen. Diese Flusslimitationsmerkmale sind in dem dargestellten Verlauf des Atemgasstromes durch darin ausgebildete Plateaus 7, sowie durch mehrere lokale Maxima 8 erkennbar. Bei den dargestellten Atemzügen treten die Flusslimitationsmerkmale jeweils in der Inspirationsphase des jeweiligen Atemzuges 1 auf. An die hier dargestellten ersten 4 Atemzüge 1 schließen sich drei zum Teil noch flusslimitierte Atemzüge 1h an die einer Hypopnoephase zuzuordnen sind und zum Teil ebenfalls noch Flusslimitationsmerkmale zeigen.

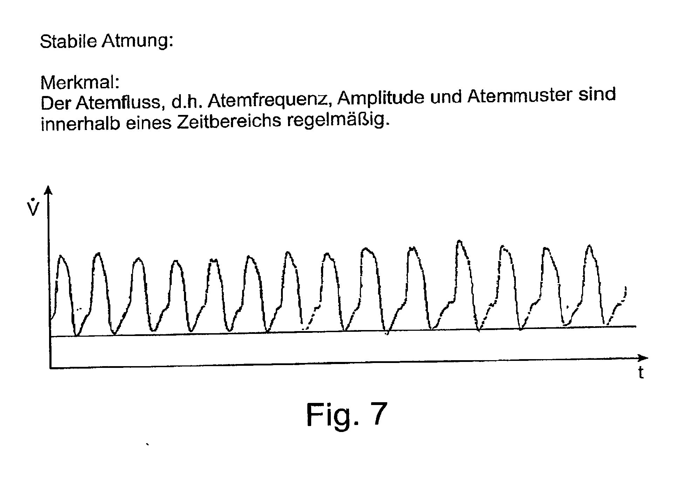

Fig. 7 zeigt den Verlauf des Atemgasstromes bei einer als stabil klassifizierten Atmungsperiode. Der Atemgasfluss, die Atemfrequenz, die Amplitude und das Atemmuster des Atemgasstromes sind innerhalb eines vorgegebenen Bereiches der als Zeitbereich oder auch durch eine Anzahl von Atemzügen definiert werden kann, regelmäßig. Die Atmungsstabilität bewegt sich bei dem hierbei dargestellten Verlauf des Atemgasstromes oberhalb eines Atmungsstabilitätsgrenzwertes von 0,86. Zusätzlich kann eine statistische Analyse der Inspirationszeit /Exspirationszeit und Gesamtatemzugszeit über mehrere Atemzüge (vorzugsweise 10 Atemzüge) erfolgen. Während der hier dargestellten Phase stabiler Atmung treten keine Atemstörungen (OSA) auf.

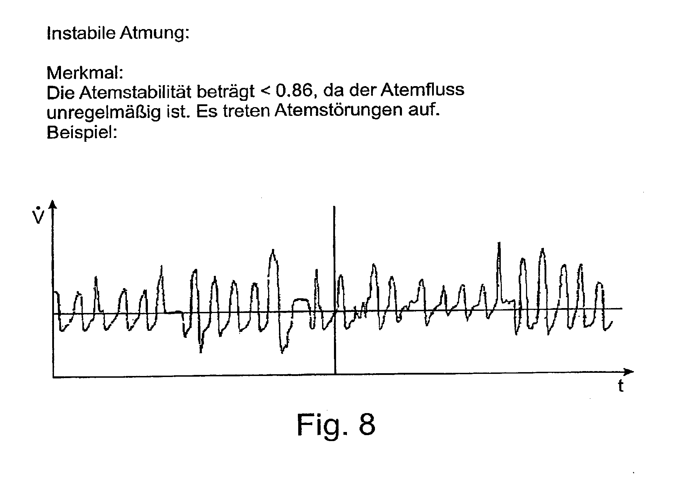

In Fig. 8 ist der zeitliche Verlauf des Atemgasstromes für mehrere Atemzüge dargestellt, wobei während des dargestellten Zeitabschnitts der Atemfluss unregelmäßig ist und bei einzelnen Atemzügen Atemstörungen (OSA) auftreten. Zusätzlich kann eine statistische Analyse der Inspirationszeit/Exspirationszeit und Gesamtatemzugszeit über mehrere Atemzüge (vorzugsweise 10 Atemzüge) erfolgen. Bei dem hier gezeigten Ausführungsbeispiel liegt der Atmungsstabilitätsindex unter dem Grenzwert von vorzugsweise 0,911.

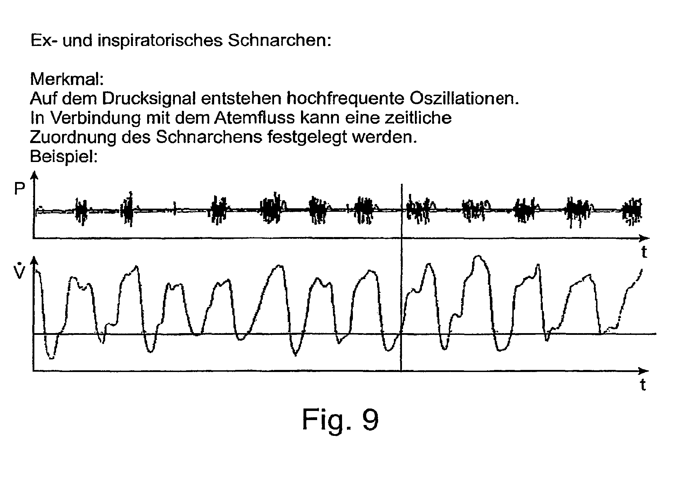

In Fig. 9 ist der zeitliche Verlauf des Atemgasstromes in Verbindung mit einem Atemgas-Drucksignal dargestellt. In dem Atemgas-Drucksignal sind phasenweise hochfrequente Oszilllationen enthalten die beim vorliegenden Beispiel inspiratorischem Schnarchen zugeordnet werden können.

## Patentansprüche

1. Mobiles Erfassungsgerät mit einer Sensoreinrichtung zur Erfassung eines hinsichtlich des Atemgasstroms indikativen Atemstromsignals; einer Spannungsversorgungseinrichtung, die als Batterieeinrichtung ausgestaltet ist, einer Zeitgebereinrichtung, einer Schnittstelleneinrichtung; und einer unter Einschluß einer Speichereinrichtung gebildeten elektronischen Datenverarbeitungseinrichtung zur Verarbeitung des hinsichtlich des zeitlichen Verlaufs des Atemstroms indikativen Atemstromsignals und von Zeitinformation, die von der Zeitgebereinrichtung aufgezeichnet wird, wobei die Datenverarbeitungseinrichtung zur Speicherung von hinsichtlich des zeitlichen Verlaufs des Atemstroms indikativen Daten, die mit der Zeitinformation verknüpft sind konfiguriert ist, wobei die Schnittstelleneinrichtung zur Übertragung der aufgezeichneten Daten auf ein externes Auswertungssystem ausgelegt ist.

2. Erfassungsgerät nach Anspruch 1, **dadurch gekennzeichnet, dass** seitens der Datenverarbeitungseinrichtung ein Datenkompressionssystem vorgesehen ist.

3. Erfassungsgerät nach wenigstens einem der Ansprüche 1 bis 2, **gekennzeichnet durch** einen ersten und/oder zweiten Druckmessanschluss, zur Erfassung eines ersten und/oder zweiten Druckmesssignals.

4. Erfassungsgerät nach wenigstens einem der Ansprüche 1 bis 3, **dadurch gekennzeichnet, dass** es zur Erfassung für die linke und rechte Nasenöffnung jeweils indikativer Atemstromsignale ausgebildet ist.

5. Erfassungsgerät nach wenigstens einem der Ansprüche 1 bis 4, gekennzeichnetdurch eine Einrichtung zur Erfassung der Thoraxweitung.

6. Erfassungsgerät nach wenigstens einem der Ansprüche 1 bis 5, **dadurch gekennzeichnet, dass** die Schnittstelleneinrichtung als USB-Schnittstelle ausgebildet ist.

7. Erfassungsgerät nach wenigstens einem der Ansprüche 1 bis 6, **dadurch gekennzeichnet, dass** die Speichereinrichtung austauschbar in dem Aufzeichnungsgerät aufgenommen ist.

8. Erfassungsgerät nach wenigstens einem der Ansprüche 1 bis 7, **dadurch gekennzeichnet, dass** die Konfiguration der Datenverarbeitungseinrichtung durch ein Datenverarbeitungsprogramm festgelegt ist, und dass dieses Datenverarbeitungsprogramm veränderbar oder wechselbar ist.

9. Erfassungsgerät nach wenigstens einem der Ansprüche 1 bis 8, **gekennzeichnet durch** Anzeigeeinrichtungen zur Anzeige der Betriebsbereitschaft oder des Funktionszustands desselben.

10. Erfassungsgerät nach wenigstens einem der Ansprüche 1 bis 9, **dadurch gekennzeichnet, dass** die Datenverarbeitungseinrichtung mit einer Kalibriereinrichtung gekoppelt ist, zur Kalibrierung des Atemstromsignals.

11. Erfassungsgerät nach wenigstens einem der Ansprüche 1 bis 10, **dadurch gekennzeichnet, dass** dieses eine Strukturkomponente aufweist, die zu einem Wiedergabegerät kompatibel ist.

12. Erfassungsgerät nach Anspruch 11, **dadurch gekennzeichnet, dass** zumindest ein Abschnitt desselben in einen Einschubschacht eines Wiedergabegeräts einführbar ist.

13. Erfassungsgerät nach Anspruch 11 oder 12, **dadurch gekennzeichnet, dass** dieses ein Basismodul und ein mit diesem koppelbares Aufzeichnungstransfermodul umfasst.

14. Erfassungsgerät nach Anspruch 13, **dadurch gekennzeichnet, dass** das Aufzeichnungsmodul als mit dem Wiedergabegerät kompatible Struktur ausgebildet ist.

15. Erfassungsgerät nach Anspruch 13 oder 14, **dadurch gekennzeichnet, dass** das Basismodul einen Batterieschacht und/oder einen Drucksensor aufweist, zur Spannungsversorgung und/oder Konvertierung des Drucksignals über das Basismodul.

16. Erfassungsgerät nach einem der Ansprüche 13 bis 15, **dadurch gekennzeichnet, dass** das Aufzeichnungsmodul die Datenverarbeitungseinrichtung umfasst, zur Aufzeichnung von hinsichtlich des zeitlichen Verlaufs der Atmung indikativen Signalen auf der Speichereinrichtung.

17. Erfassungsgerät nach einem der Ansprüche 13 bis 16, **dadurch gekennzeichnet, dass** das Aufzeichnungsmodul eine Schnittstelleneinrichtung und/oder eine lösbar mit dem Aufzeichnungsmodul gekoppelte Speichereinrichtung aufweist.

18. Verfahren zur Bereitstellung eines hinsichtlich des physiologischen Zustands indikativen Auswertungsresultats auf der Grundlage von Messsignalen die mit der Atmung einer Person im Zusammenhang stehen, wobei aus den genannten Messsignalen unter Heranziehung mehrerer Auswertungssysteme, Auswertungsmerkmale generiert werden und im Rahmen eines hierauf basierenden Resultat-Generierungsschrittes wenigstens ein Auswertungsresultat generiert wird, indem die Auswertungsmerkmale einer verknüpfenden Betrachtung unterzogen werden, wobei die Messsignale im Rahmen einer der Auswertung vorangehenden Signalerfassungsphase durch ein mobiles Erfassungsgerät erfasst werden, wobei das mobile Erfassungsgerät eine Spannungsversorgungseinrichtung aufweist, die als Batterieeinrichtung ausgestaltet ist, wobei das mobile Erfassungsgerät ausgelegt ist, die Messsignale zu erfassen und abzuspeichern und eine Schnittstelleneinrichtung des mobilen Erfassungsgeräts ausgelegt ist, die Messsginale auf ein externes Auswertungssystem zur Generierung der Auswertungsmerkale zu übertagen, wobei der Resultat-Generierungsschritt-Beurteilungsgrundlagen für eine Prognose atembezogener Erkrankung bereitstellt.

19. Verfahren nach Anspruch 18, **dadurch gekennzeichnet, dass** ein überwiegend in den Auswertungsmerkmalen enthaltener Merkmalsbeitrag innerhalb eines Generierungs-Zeitfensters generiert wird das kleiner ist als ein für die verknüpfende Betrachtung vorgesehenes Verknüpfungszeitfenster.

20. Verfahren nach wenigstens einem der Ansprüche 18 bis 19, **dadurch gekennzeichnet, dass** die generierten Auswertungsmerkmale unter Zuordnung ihrer zeitlichen Lage in dem Messsignalerhebungszeitraum gespeichert werden.

21. Verfahren nach wenigstens einem der Ansprüche 18 bis 20, **dadurch gekennzeichnet, dass** durch ein Titrationsverfahren Auswertungsergebnisse generiert werden, die eine Klassifizierung oder Beurteilung eines Anwenders hinsichtlich obstruktiver, zentraler und/oder gemischter Atmungsstörungen oder die Abgabe einer Therapieempfehlung ermöglichen.

22. Verfahren nach wenigstens einem der Ansprüche 18 bis 21, **dadurch gekennzeichnet, dass** das mobile Erfassungsgerät ein mobiles Erfassungsgerät nach einem der Ansprüche 1 bis 17 ist.

## Claims

1. A mobile detection appliance with a sensor device for acquiring a respiratory flow signal that is indicative of the respiratory gas flow, a power supplying device which is realized as a battery device, a time-keeping device, an interface device and an electronic data processing unit which comprises a memory device and is used to process the respiratory flow signal that is indicative of the temporal course of the respiratory flow and time information recorded by the time-keeping device, said data processing unit being configured to store data that are indicative of the temporal course of the respiratory flow and that are associated with the time information, wherein the interface device is adapted to transmit the recorded data to an external analysis system.

2. The detection appliance according to claim 1, **characterized in that** the data processing unit is provided with a data compression system.

3. The detection appliance according to at least one of the claims 1 to 2, **characterized by** a first and/or a second pressure measurement connection for measuring a first and/or a second pressure measurement signal.

4. The detection appliance according to at least one of the claims 1 to 3, **characterized in that** it is designed to acquire separate respiratory flow signals that are indicative of the left and right nasal openings respectively.

5. The detection appliance according to at least one of the claims 1 to 4, **characterized by** a device for detecting the chest expansion.

6. The detection appliance according to at least one of the claims 1 to 5, **characterized in that** the interface device is in the form of a USB interface.

7. The detection appliance according to at least one of the claims 1 to 6, **characterized in that** the memory device is removably incorporated in the recording device.

8. The detection appliance according to at least one of the claims 1 to 7, **characterized in that** the configuration of the data processing unit is set by a data processing program, said data processing program being modifiable or substitutable.

9. The detection appliance according to at least one of the claims 1 to 8, **characterized by** indicator devices for indicating the operational readiness or the functional state thereof.

10. The detection appliance according to at least one of the claims 1 to 9, **characterized in that** the data processing unit is coupled to a calibration device for calibrating the respiratory flow signal.

11. The detection appliance according to at least one of the claims 1 to 10, **characterized in that** said detection appliance exhibits a structural component that is compatible with a playback unit.

12. The detection appliance according to claim 11, **characterized in that** at least one portion of said detection appliance can be introduced into an insertion slot of a playback unit.

13. The detection appliance according to claim 11 or 12, **characterized in that** said detection appliance comprises a base module and a recording transfer module which can be coupled therewith.

14. The detection appliance according to claim 13, **characterized in that** the recording module is configured as a structure being compatible with the playback unit.

15. The detection appliance according to claim 13 or 14, **characterized in that** the base module comprises a battery slot and/or a pressure sensor for power supply and/or conversion of the pressure signal via the base module.

16. The detection appliance according to one of the claims 13 to 15, **characterized in that** the recording module comprises the data processing unit for recording signals that are indicative of the temporal course of the respiration on the memory device.

17. The detection appliance according to one of the claims 13 to 16, **characterized in that** the recording module comprises an interface device and/or a memory device being detachably coupled with the recording module.

18. A method for the provision of an evaluation result that is indicative of the physiological state and is based on measurement signals associated with the respiration of a person, where evaluation characteristics are generated from said measurement signals through the use of several analysis systems and at least one evaluation result is generated from a result generation step based thereon in which the evaluation characteristics are subjected to an associative analysis, wherein said measurement signals are acquired by a mobile detection appliance in the course of a signal acquisition phase preceding the analysis, wherein the mobile detection appliance comprises a power supply means which is realized as a battery device, wherein the mobile detection appliance is adapted to detect and store the measurment signals and an interface device of the mobile detection appliance is adapted to transmit the measurment signals to an external evaluation system for generating the evaluation characteristics, wherein the result generation step provides an assessment basis for the prognosis of respiratory related illnesses.

19. The method according to claim 18, **characterized in that** a contributing characteristic predominantly included among the evaluation characteristics is generated within a generation time window that is smaller than an association time window used in the associative analysis.

20. The method according to at least one of the claims 18 to 19, **characterized in that** the generated evaluation characteristics are stored in conjunction with the time position within the measurement signal acquisition period.

21. The method according to at least one of the claims 18 to 20, **characterized in that** a titration method is used to generate evaluation results that enable a classification or assessment of a user in regard to obstructive, central and/or mixed respiratory disorders or the provision of a therapy recommendation.

22. The method according to at least one of the claims 18 to 21, **characterized in that** the mobile detection appliance is a mobile detection appliance according to one of claims 1 to 17.

## Revendications

1. Appareil de détection mobile comportant un dispositif de capteur pour la détection d'un signal de flux respiratoire caractéristique pour le flux de gaz respiratoire, un dispositif d'alimentation en tension réalisé comme dispositif à accumulateur, un dispositif de temporisation, un dispositif d'interface ; et un dispositif électronique de traitement des données formé en intégrant un dispositif de mémorisation pour le traitement du signal de flux respiratoire caractéristique pour la courbe temporelle du flux respiratoire et d'une information temporelle enregistrée par le dispositif de temporisation, le dispositif de traitement des données étant configuré pour la mémorisation de données caractéristiques pour la courbe temporelle du flux respiratoire, lesquelles sont associées à l'information temporelle, le dispositif d'interface étant prévu pour la transmission des données enregistrées à un système d'évaluation extérieur.

2. Appareil de détection selon la revendication 1, **caractérisé en ce qu'**un système de compression des données est prévu pour le dispositif de traitement des données.

3. Appareil de détection selon la revendication 1 ou la revendication 2, **caractérisé par** un premier et/ou un deuxième accord de mesure de pression pour la détection d'un premier et/ou un d'un deuxième signal de mesure de pression.

4. Appareil de détection selon au moins une des revendications 1 à 3, **caractérisé en ce qu'**il est prévu pour la détection de signaux de flux respiratoire caractéristiques pour chacune de la narine gauche et la narine droite.

5. Appareil de détection selon au moins une des revendications 1 à 4, **caractérisé par** un dispositif de détermination de l'ampliation thoracique.

6. Appareil de détection selon au moins une des revendications 1 à 5, **caractérisé en ce que** le dispositif d'interface est réalisé sous forme de port USB.

7. Appareil de détection selon au moins une des revendications 1 à 6, **caractérisé en ce que** le dispositif de mémorisation est mis en place de manière remplaçable dans l'appareil enregistreur.

8. Appareil de détection selon au moins une des revendications 1 à 7, **caractérisé en ce que** la configuration du dispositif de traitement des données est fixée par un programme de traitement des données, et **en ce que** ledit programme de traitement des données peut être modifié ou changé.

9. Appareil de détection selon au moins une des revendications 1 à 8, **caractérisé par** des dispositifs indicateurs pour l'indication de l'aptitude au fonctionnement ou de l'état de service.

10. Appareil de détection selon au moins une des revendications 1 à 9, **caractérisé en ce que** le dispositif de traitement des données est couplé à un dispositif de calibrage pour le calibrage du signal de flux respiratoire.

11. Appareil de détection selon au moins une des revendications 1 à 10, **caractérisé en ce qu'**il comporte une composante structurelle compatible avec un appareil de lecture.

12. Appareil de détection selon la revendication 11, **caractérisé en ce qu'**au moins une partie de celui-ci est insérable dans une fente d'insertion d'un appareil de lecture.

13. Appareil de détection selon la revendication 11 ou la revendication 12, **caractérisé en ce qu'**il comprend un module de base et un module de transfert d'enregistrement pouvant être couplé à celui-ci.

14. Appareil de détection selon la revendication 13, **caractérisé en ce que** le module d'enregistrement est réalisé comme structure compatible avec l'appareil de lecture.

15. Appareil de détection selon la revendication 13 ou la revendication 14, **caractérisé en ce que** le module de base présente un compartiment d'accumulateur et/ou un capteur de pression pour l'alimentation en tension et/ou la conversion du signal de pression par le module de base.

16. Appareil de détection selon l'une des revendications 13 à 15, **caractérisé en ce que** le module d'enregistrement comprend le dispositif de traitement des données pour l'enregistrement dans le dispositif de mémoire de signaux indicateurs pour la courbe temporelle de la respiration.

17. Appareil de détection selon l'une des revendications 13 à 16, **caractérisé en ce que** le module d'enregistrement comporte un dispositif d'interface et/ou un dispositif de mémorisation couplé de manière amovible au module d'enregistrement.

18. Procédé d'élaboration d'un résultat d'évaluation caractéristique pour l'état physiologique sur la base de signaux de mesure en relation avec la respiration d'une personne, où des caractéristiques d'évaluation sont générées à partir des signaux de mesure susmentionnés en recourant à plusieurs systèmes d'évaluation, et où au moins un résultat d'évaluation est généré lors d'une étape de génération de résultat basée sur celles-ci, les caractéristiques d'évaluation étant soumises à une analyse associative, les signaux de mesure étant détectés lors d'une phase de détection de signaux antérieure à l'évaluation par un appareil de détection mobile, ledit appareil de détection mobile comportant un dispositif d'alimentation en tension réalisé comme dispositif à accumulateur, ledit appareil de détection mobile étant prévu pour détecter et mémoriser les signaux de mesure, et un dispositif d'interface de l'appareil de détection mobile étant prévu pour transmettre les signaux de mesure à un système d'évaluation extérieur pour générer les caractéristiques d'évaluation, l'étape de génération de résultat élaborant des bases d'appréciation pour un diagnostic de pathologies respiratoires.

19. Procédé selon la revendication 18, **caractérisé en ce qu'**une composante de caractéristique majoritairement contenue dans les caractéristiques d'évaluation est générée à l'intérieur d'une fenêtre temporelle de génération de grandeur inférieure à une fenêtre temporelle d'association prévue pour l'analyse associative.

20. Procédé selon au moins une des revendications 18 ou 19, **caractérisé en ce que** les caractéristiques d'évaluation générées sont mémorisées avec classement de leur position temporelle dans la période de prélèvement de signal de mesure.

21. Procédé selon au moins une des revendications 18 à 20, **caractérisé en ce que** des résultats d'évaluation sont générés au moyen d'un procédé de titrage, lesquels permettent une classification ou une évaluation d'un utilisateur pour des troubles respiratoires obstructifs, centraux et/ou mixtes, ou l'émission d'une recommandation thérapeutique.

22. Procédé selon au moins une des revendications 18 à 21, **caractérisé en ce que** l'appareil de détection mobile est un appareil de détection mobile selon l'une des revendications 1 à 17.
